# EUROPÄISCHE PATENTSCHRIFT

(11) **EP 2 089 525 B1**
(45) Veröffentlichungstag und Bekanntmachung des Hinweises auf die Patenterteilung: **16.03.2016**
(21) Anmeldenummer: 07821770.0
(22) Anmeldetag: 24.10.2007
(51) Int. Cl.: C12N 15/77, C12P 13/08, C12P 13/22, A23K 10/40, C12R 1/15

(54) **ALLELE DES OXYR-GENS AUS CORYNEFORMEN BAKTERIEN**
ALLELS OF THE OXYR GENE OF CORYNEFORM BACTERIA
ALLÈLES DU GÈNE OXYR DE BACTÉRIES CORYNÉFORMES

(30) Priorität: 17.11.2006 DE 102006054202
(43) Veröffentlichungstag der Anmeldung: 19.08.2009
(73) Patentinhaber: Evonik Degussa GmbH, 45128 Essen (DE)
(72) Erfinder: BATHE, Brigitte, 33154 Salzkotten (DE); CLAES, Wilfried, 33615 Bielefeld (DE); JERRENTRUP, Silke, 33607 Bielefeld (DE); KREUTZER, Caroline, 33813 Oerlinghausen (DE); THIERBACH, Georg, 33613 Bielefeld (DE)
(86) Internationale Anmeldenummer: PCT/EP2007/061407
(87) Internationale Veröffentlichungsnummer: WO 2008/058838

(56) Entgegenhaltungen:
- EP-B- 0 615 693
- EP-B- 1 029 919
- WO-A-02/18431
- WO-A-2005/083082
- KALINOWSKI J ET AL: "THE COMPLETE CORYNEBACTERIUM GLUTAMICUM ATCC 13032 GENONE SEQUENCE AND ITS IMPACT ON THE PRODUCTION OF L-ASPARTATE-DERIVED AMINO ACIDS AND VITAMINS" JOURNAL OF BIOTECHNOLOGY, ELSEVIER SCIENCE PUBLISHERS B.V., AMSTERDAM, NL, Bd. 104, Nr. 1-3, 4. September 2003 (2003-09-04), Seiten 5-25, XP001184752 ISSN: 0168-1656
- YUKAWA HIDEAKI ET AL: "Comparative analysis of the Corynebacterium glutamicum group and complete genome sequence of strain R" MICROBIOLOGY (READING), Bd. 153, Nr. Part 4, April 2007 (2007-04), Seiten 1042-1058, XP002472798 ISSN: 1350-0872

## Beschreibung

Gegenstand der Erfindung sind Mutanten und Allele des oxyR-Gens coryneformer Bakterien kodierend für Varianten des OxyR-Transkriptionsregulators und Verfahren zur Herstellung von L-Lysin unter Verwendung von Bakterien, die diese Allele enthalten.

### Stand der Technik

Aminosäuren finden in der Humanmedizin, in der pharmazeutischen Industrie, in der Lebensmittelindustrie und ganz besonders in der Tierernährung Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die AminosäureProduktion untersucht.

Das Chromosom von Corynebacterium glutamicum wurde vor einiger Zeit vollständig sequenziert (Kalinowski et al., Journal of Biotechnology 104, 5-25 (2003)). Das Chromosom von Corynebacterium efficiens wurde ebenfalls bereits sequenziert (Nishio et al., Genome Res. 13 (7), 1572-1579 (2003)).

Entsprechende Sequenzangaben können den öffentlichen Datenbanken entnommen werden. Geeignete Datenbanken sind beispielsweise die Datenbank der European Molecular Biologies Laboratories (EMBL, Heidelberg, Deutschland bzw. Cambridge, UK), die Datenbank des National Center for Biotechnology Information (NCBI, Bethesda, MD, USA), die des Swiss Institute of Bioinformatics (Swissprot, Geneve, Switzerland), die Protein Information Resource Database (PIR, Washington, DC, USA) und die DNA Data Bank of Japan (DDBJ, 1111 Yata, Mishima, 411-8540, Japan).

Zusammenfassende Darstellungen zur Genetik, zum Stoffwechsel und zur technischen Bedeutung von Corynebacterium findet man in den Aufsätzen von Ikeda, von Pfefferle et al. und von Mueller und Huebner im Buch "Microbial Production of L-Amino Acids" (Advances in Biochemical Engineering 79, (2003), Springer Verlag, Berlin, Deutschland, Herausgeber: T. Scheper), in der Spezialausgabe "A New Era in Corynebacterium glutamicum Biotechnology" des Journal of Biotechnology (Band 104 (1-3), 2003, Herausgeber: A. Pühler und T. Tauch) und im "Handbook of Corynebacterium glutamicum" (Herausgeber: L. Eggeling und M. Bott, CRC Press, Taylor & Francis Group, Boca Raton, FL, USA, 2005).

Die Nukleotidsequenz des für den OxyR-Transkriptionsregulator von Corynebacterium glutamicum kodierenden oxyR-Gens ist unter anderem in der Datenbank des National Center for Biotechnology Information (NCBI) der National Library of Medicin (Bethesda, MD, USA) unter der Zugangsnummer AR697215 allgemein verfügbar. Sie kann weiterhin der Patentanmeldung EP1108790 als Sequenz Nr. 2114 entnommen werden.

In der US 6,916,636 wird über die Auswirkung der Überexpression des OxyR-Transkriptionsregulators auf die Aminosäureproduktion berichtet.

In der WO 2002/018431 wird die verbesserte Produktion von L-Aminosäuren, unter anderem L-Lysin, bei Überexpression des oxyR-Gens in Corynebacterium glutamicum, offenbart.

Die WO 2005/083082 offenbart ebenfalls die Überexpression des oxyR-Gens zur verbesserten Produktion von L-Aminosäuren in coryneformen Bakterien.

Der besseren Übersichtlichkeit halber ist die Nukleotidsequenz des für den OxyR-Transkriptionsregulator des Wildtyps von Corynebacterium glutamicum kodierenden oxyR-Gens ("Wildtyp-Gen") gemäß den Angaben der NCBI-Datenbank in SEQ ID NO:1 und die sich daraus ergebende Aminosäuresequenz des kodierten OxyR-Transkriptionsregulators in SEQ ID NO:2 bzw. 4 dargestellt. In SEQ ID NO:3 sind stromaufwärts (upstream) und stromabwärts (downstream) gelegene Nukleotidsequenzen zusätzlich angegeben.

### Aufgabe der Erfindung

Die Erfinder haben sich zur Aufgabe gestellt neue Maßnahmen zur verbesserten Herstellung von L-Lysin bereitzustellen.

### Beschreibung der Erfindung

Gegenstand der Erfindung sind erzeugte bzw. isolierte Mutanten coryneformer Bakterien welche ein Gen bzw. Allel enthalten, das für ein Polypeptid mit OxyR-Transkriptionsregulator-Aktivität kodiert, dadurch gekennzeichnet, dass das Polypeptid eine Aminosäuresequenz umfasst, ausgewählt aus der Gruppe:
a) Aminosäuresequenz SEQ ID NO: 6, die durch Austausch von L-Alanin gegen L-Valin an der Position 89 in SEQ ID NO: 2 entstanden ist oder
b) Aminosäuresequenz gemäß SEQ ID NO: 6, die durch Austausch von L-Alanin gegen L-Valin an der Position 89 in SEQ ID NO: 2 entstanden ist und die zusätzlich maximal fünf konservative Aminosäureaustausche und maximal 5 Insertionen oder Deletionen enthält, wobei die maximal fünf konservativen Aminosäureaustausche und maximal 5 Insertionen oder Deletionen die Aktivität im Wesentlichen nicht verändern.

Es ist bekannt, dass konservative Aminosäureaustausche die Aktivität nur unwesentlich verändern. Dementsprechend kann das in den erfindungsgemäßen Mutanten enthaltene oxyR-Allel, das für ein Polypeptid mit OxyR-Transkriptionsregulator-Aktivität kodiert, zusätzlich zu der in SEQ ID NO:6 gezeigten Aminosäuresequenz einen (1) oder mehrere konservative Aminosäureaustausch(e) enthalten. Bevorzugt enthält das Polypeptid höchstens fünf (5) konservative Aminosäureaustausche.

Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Durch Vergleich der Aminosäuresequenz mit dem Clustal-Programm (Thompson et al., Nucleic Acids Research 22, 4637-4680 (1994)) wurde festgestellt, dass die Aminosäuresequenzen der OxyR-Transkriptionsregulatoren verschiedener Bakterien wie bespielsweise Escherichia coli, Streptomyces coelicolor, Streptomyces avermitilis, Corynebacterium efficiens und Corynebacterium glutamicum ein Sequenzmotiv bestehend aus der Abfolge Leu-Gly-Val-Met/Thr/Gln-Leu-Ile/Leu-Glu-Arg-Thr/Ser-Thr/Ser-Arg-Lys-Val-Ile/Leu, ein Sequenzmotiv bestehend aus der Abfolge Ile-Pro-Thr-Val/Ala-Ala/Gly-Pro-Tyr-Ile/Leu-Leu-Pro und auch ein Sequenzmotiv bestehend aus der Abfolge Leu-Leu-Leu/Met-Leu-Glu/Asp-Glu/Asp-Gly-His-Cys-Leu-Arg/His-Asp-Gln enthalten. Die Bezeichnungen "Met/Thr/Gln", "Ile/Leu", "Thr/Ser", "Val/Ala", "Ala/Gly", "Leu/Met", "Glu/Asp", und "Arg/His" bedeuten, dass an der entsprechenden Position "Met oder Thr oder Gln", "Ile oder Leu", "Thr oder Ser", "Val oder Ala", "Ala oder Gly", "Leu oder Met", "Glu oder Asp", oder "Arg oder His" enthalten sind.

Das Aminosäuresequenzmotiv Leu-Gly-Val-Met/Thr/Gln-Leu-Ile/Leu-Glu-Arg-Thr/Ser-Thr/Ser-Arg-Lys-Val-Ile/Leu ist beispielsweise in der SEQ ID NO:2 bzw. 4 oder 6 bzw. 8 von Position 50 bis 63 enthalten. Das Aminosäuresequenzmotiv Ile-Pro-Thr-Val/Ala-Ala/Gly-Pro-Tyr-Ile/Leu-Leu-Pro ist beispielsweise in der SEQ ID NO:2 bzw. 4 oder 6 bzw. 8 von Position 105 bis 114 enthalten. Das Aminosäuresequenzmotiv Leu-Leu-Leu/Met-Leu-Glu/Asp-Glu/Asp-Gly-His-Cys-Leu-Arg/His-Asp-Gln ist beispielsweise in der SEQ ID NO: 2 bzw. 4 oder 6 bzw. 8 von Position 198 bis 210 enthalten.

"Im Wesentlichen nicht berührt" bedeutet, dass sich die Aktivität der genannten Varianten um maximal 10% von der Aktivität des Polypeptids mit der Aminosäuresequenz von SEQ ID NO:6 unterscheidet. (Von S. 15, Zeilen 23-28)

Die zuvor genannten Sequenzmotive Leu-Gly-Val-Met/Thr/Gln-Leu-Ile/Leu-Glu-Arg-Thr/Ser-Thr/Ser-Arg-Lys-Val-Ile/Leu, Ile-Pro-Thr-Val/Ala-Ala/Gly-Pro-Tyr-Ile/Leu-Leu-Pro und Leu-Leu-Leu/Met-Leu-Glu/Asp-Glu/Asp-Gly-His-Cys-Leu-Arg/His-Asp-Gln werden durch derartige Insertionen/Deletionen vorzugsweise nicht zerrissen.

Bei den coryneformen Bakterien wird die Gattung Corynebacterium bevorzugt. Bei der Gattung Corynebacterium werden folgenden Arten als Ausgangsstämme bevorzugt:
Corynebacterium efficiens (Typ-Stamm DSM44549),
Corynebacterium glutamicum (Typ-Stamm ATCC13032),
Corynebacterium thermoaminogenes (beispielsweise der Stamm FERM BP-1539), und
Corynebacterium ammoniagenes (Typ-Stamm ATCC6871),
wobei die Art Corynebacterium glutamicum ganz besonders bevorzugt wird.

Einige Vertreter der Art Corynebacterium glutamicum sind im Stand der Technik auch unter anderen Artbezeichnungen bekannt. Hierzu gehören beispielsweise:
Corynebacterium acetoacidophilum ATCC13870,
Corynebacterium lilium DSM20137,
Corynebacterium melassecola ATCC17965,
Brevibacterium flavum ATCC14067,
Brevibacterium lactofermentum ATCC13869,
Brevibacterium divaricatum ATCC14020, und
Microbacterium ammoniaphilum ATCC15354.

Der Begriff "Micrococcus glutamicus" für Corynebacterium glutamicum war ebenfalls gebräuchlich.

Die für die Maßnahmen der Erfindung eingesetzten Stämme coryneformer Bakterien (Ausgangsstämme) besitzen bevorzugt bereits die Fähigkeit, L-Lysin in der Zelle anzureichern oder in das sie umgebende Nährmedium auszuscheiden und zu akkumulieren. Hierfür wird im Folgenden auch der Ausdruck "produzieren" verwendet. Insbesondere besitzen die für die Maßnahmen der Erfindung eingesetzten Stämme coryneformer Bakterien die Fähigkeit ≥ (mindestens) 0,25 g/l, ≥ 0,5 g/l, ≥ 1,0 g/1, ≥ 1,5 g/l, ≥ 2,0 g/l, ≥ 4 g/l oder ≥ 10 g/l der gewünschten Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Bekannte Vertreter L-Lysin produzierender bzw. ausscheidender Stämme coryneformer Bakterien sind beispielsweise:
Corynebacterium glutamicum DM58-1/pDM6 (= DSM4697) beschrieben in EP 0 358 940,
Corynebacterium glutamicum MH20-22B (= DSM16835) beschrieben in Menkel et al. (Applied and Environmental Microbiology 55(3), 684-688 (1989)),
Corynebacterium glutamicum AHP-3 (= Ferm BP-7382) beschrieben in EP 1 108 790,
Corynebacterium glutamicum NRRL B-11474 beschrieben in US 4,275,157, und
Corynebacterium thermoaminogenes AJ12521 (= FERM BP-3304) beschrieben in US 5,250,423.

Angaben zur taxonomischen Einordnung von Stämmen dieser Gruppe von Bakterien findet man unter anderem bei Seiler (Journal of General Microbiology 129, 1433-1477 (1983)), Kinoshita (1985, Glutamic Acid Bacteria, p 115-142. In: Demain and Solomon (ed), Biology of Industrial Microorganisms. The Benjamin/Cummins Publishing Co., London, UK), Kämpfer und Kroppenstedt (Canadian Journal of Microbiology 42, 989-1005 (1996)), Liebl et al (International Journal of Systematic Bacteriology 41, 255-260 (1991)) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "NRRL" können von der Agricultural Research Service Patent Culture Collection (ARS, Peoria, Illinois, US) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden.

Ein Gen oder Allel ist chemisch ein Polynukleotid. Ein anderer Begriff hierfür ist Nukleinsäure.

Das vom oxyR-Gen von Corynebacterium glutamicum kodierte Polypeptid mit OxyR-Transkriptionsregulator-Aktivität gehört zur Gruppe der sogenannten LysR-Typ Transkriptionsregulatoren (LTTR). Eine Übersicht über LTTR's findet man bei Schell (Annual Reviews in Microbiology 47, 597-626 (1993)).

Der OxyR-Transkriptionsregulator kann auch als OxyR-Polypeptid, OxyR-Protein oder OxyR-Regulatorprotein bezeichnet werden. Er hat die Aktivität eines DNA-Bindeproteins und bindet im Promotorbereich des dps-Gens, welches für das starvation-induced DNA protecting Protein (Dps) kodiert. Die Nukleotidsequenz des dps-Gens von Corynebacterium glutamicum ist in SEQ ID NO:23 dargestellt. Bevorzugt bindet das OxyR-Polypeptid an die Nukleotidsequenz zwischen Position 80 und 210, besonders bevorzugt zwischen 110 und 210 und ganz besonders bevorzugt an die Nukleotidsequenz zwischen Position 167 und 179 von SEQ ID NO:23.

Der Nachweis der Bindung erfolgt mit Hilfe eines Testes, der in der Fachwelt als "electrophoretic mobility shift analysis"-Test (EMSA) bekannt ist und beispielsweise bei Kerr beschrieben ist (Methods in Enzymology, 254:619-32 (1995)). Hierbei wird das zu untersuchende DNA-Fragment mittels PCR amplifiziert und durch die Verwendung von modifizierten Primern, die ein Fluorophor am 5'-Ende des Primers tragen, markiert. Als Fluorophor wird z.B. das Indocarbocyanine (Cy3) verwendet. Die markierte DNA wird mit gereinigtem OxyR-Regulatorprotein gemischt und die Wirkung der Gegenwart des Proteins auf die Mobilität des DNA-Fragments im elektrischen Feld mittels Agarosegelelektrophorese gefolgt von Fluoreszenzdetektion analysiert. Weitere methodische Details zum EMSA-Test kann man unter anderem bei Rey et al (Molecular Microbiology 56(4), 871-887 (2005) nachlesen.

Die erfindungsgemäßen Mutanten scheiden bevorzugt L-Lysin aus. Der Begriff L-Lysin umfasst auch dessen Salze wie beispielsweise das Lysin-Monohydrochlorid oder Lysin.

Gegenstand der Erfindung sind weiterhin Mutanten coryneformer Bakterien, die ein oxyR-Allel enthalten, das für ein Polypeptid mit OxyR-Transkriptionsregulator-Aktivität kodiert, das die Aminosäuresequenz SEQ ID NO:6 enthält. Bevorzugt umfasst die Länge des kodierten Polypeptids 327 Aminosäuren.

Gegenstand der Erfindung sind weiterhin Mutanten coryneformer Bakterien, die ein oxyR-Allel enthalten, das für ein Polypeptid mit OxyR-Transkriptionsregulator-Aktivität kodiert, welches die Nukleotidsequenz von SEQ ID NO:5 oder SEQ ID NO: 7 umfasst, wobei an Position 731 von SEQ ID NO: 5 bzw. an Position 1481 von SEQ ID NO: 7 jeweils Thymin anstelle von Cytosin enthalten ist.

Es ist bekannt, dass durch wirtseigene Enzyme, sogenannte Aminopeptidasen, das endständige Methionin bei der Proteinsynthese entfernt wird.

Zur Herstellung der erfindungsgemäßen Mutanten können klassische in-vivo Mutageneseverfahren mit Zellpopulationen coryneformer Bakterien unter Verwendung mutagener Stoffe wie beispielsweise N-Methyl-N'-Nitro-N-Nitrosoguanidin (MNNG), Ethylmethansulfonat (EMS), 5-Bromuracil oder ultraviolettes Licht verwendet werden. Mutagenesemethoden sind beispielsweise im Manual of Methods for General Bacteriology (Gerhard et al. (Eds.), American Society for Microbiology, Washington, DC, USA, 1981) oder bei Tosaka et al. (Agricultural and Biological Chemistry 42(4), 745-752 (1978)) oder bei Konicek et al. (Folia Microbiologica 33, 337-343 (1988)) beschrieben. Typische Mutagenesen unter Verwendung von MNNG umfassen Konzentrationen von 50 bis 500 mg/l oder auch höhere Konzentrationen bis zu maximal 1 g/l, eine Inkubationszeit von 1 bis 30 Minuten bei einem pH von 5,5 bis 7,5. Unter diesen Bedingungen wird die Zahl der lebensfähigen Zellen um einen Anteil von ca. 50% bis 90% oder ca. 50% bis 99% oder ca. 50% bis 99,9% oder mehr reduziert.

Aus der mutagenisierten Zellpopulation werden Mutanten bzw. Zellen entnommen und vermehrt. Vorzugsweise wird in einem weiteren Schritt deren Fähigkeit untersucht, in einer Satzkultur (batch) unter Verwendung eines geeigneten Nährmediums bevorzugt L-Lysin auszuscheiden. Geeignete Nährmedien und Testbedingungen sind unter anderem in der US 6,221,636, in der US 5,840,551, in der US 5,770,409, in der US 5,605,818, in der US 5,275,940 und in der US 4,224,409 beschrieben. Bei Verwendungen von geeigneten Roboteranlagen, wie beispielsweise bei Zimmermann et al. (VDI Berichte Nr. 1841, VDI-Verlag, Düsseldorf, Deutschland 2004, 439-443) oder Zimmermann (Chemie Ingenenieur Technik 77 (4), 426-428 (2005))beschrieben, können zahlreiche Mutanten in kurzer Zeit untersucht werden. Im Allgemeinen werden maximal 3.000, maximal 10.000, maximal 30.000 oder auch maximal 60.000 Mutanten gegebenenfalls auch mehr untersucht. Auf diese Weise werden Mutanten identifiziert, die im Vergleich zum Elternstamm bzw. nicht mutagenisierten Ausgangsstamm vermehrt L-Lysin in das Nährmedium oder in das Zellinnere ausscheiden. Dazu gehören beispielsweise solche Mutanten, deren L-Lysin-Sekretion um mindestens 0,5% erhöht ist.

Anschließend wird aus den Mutanten DNA bereitgestellt, bzw. isoliert und mit Hilfe der Polymerase-Kettenreaktion unter Verwendung von Primerpaaren, die die Amplifizierung des oxyR-Gens bzw. des erfindungsgemäßen oxyR-Allels oder der erfindungsgemäßen Mutation an Position 89 erlauben, das entsprechende Polynukleotid synthetisiert. Vorzugsweise wird die DNA aus solchen Mutanten isoliert, die in erhöhter Weise L-Lysin ausscheiden.

Hierzu können beliebige Primerpaare aus der stromaufwärts und stromabwärts der erfindungsgemäßen Mutation gelegenen Nukleotidsequenz und der dazu komplementären Nukleotidsequenz ausgewählt werden. Ein Primer eines Primerpaares umfasst hierbei bevorzugt mindestens 15, mindestens 18, mindestens 20, mindestens 21 oder mindestens 24 aufeinanderfolgende Nukleotide ausgewählt aus der Nukleotidsequenz zwischen Position 1 und 1014 von SEQ ID NO:3 oder SEQ ID NO:7. Der dazugehörige zweite Primer eines Primerpaares umfasst mindestens 15, mindestens 18, mindestens 20, mindestens 21 oder mindestens 24 aufeinanderfolgende Nukleotide ausgewählt aus der komplementären Nukleotidsequenz von Position 2484 und 1018 von SEQ ID NO:3 oder SEQ ID NO:7.

Ist die Amplifikation der Kodierregion gewünscht, so wird das Primerpaar vorzugsweise aus der Nukleotidsequenz zwischen Position 1 und 750 von SEQ ID NO:3 oder SEQ ID NO:7 und aus der komplementären Nukleotidsequenz zwischen Position 2484 und 1732 von SEQ ID NO:3 oder SEQ ID NO:7 ausgewählt. Ein geeignetes Primerpaar ist beispielsweise das unter SEQ ID NO:9 und SEQ ID NO:10 wiedergegebene Primerpaar oxyR_XL_A1 und oxyR_XL_E1.

Ist die Amplifikation eines Teils der Kodierregion, wie beispielsweise in SEQ ID NO:17 und 18 dargestellt, erwünscht, so wird das Primerpaar vorzugsweise aus der Nukleotidsequenz zwischen Position 751 und 1014 oder zwischen Position 1 und 1014 von SEQ ID NO:3 oder SEQ ID NO:7 und aus der komplementären Nukleotidsequenz zwischen Position 1731 und 1018 oder 2484 und 1018 von SEQ ID NO:3 oder SEQ ID NO:7 ausgewählt. Der Primer kann überdies mit Erkennungsstellen für Restriktionsenzyme, mit einer Biotin-Gruppe oder weiteren Accessoirs, wie sie im Stand der Technik beschrieben sind, ausgerüstet sein. Die Gesamtlänge des Primers beträgt im Allgemeinen maximal 30, 40, 50 oder 60 Nukleotide.

Für die Herstellung von Polynukleotiden durch Amplifikation ausgewählter Sequenzen, wie dem erfindungsgemäßen oxyR-Allel, aus vorgelegter, beispielsweise chromosomaler DNA ("template-DNA") durch Amplifikation mittels PCR, werden im Allgemeinen thermostabile DNA-Polymerasen eingesetzt. Beispiele derartiger DNA-Polymerasen sind die Taq-Polymerase aus Thermus aquaticus, die unter anderem von der Firma Qiagen (Hilden, Deutschland) vertrieben wird, die Vent-Polymerase aus Thermococcus litoralis, die unter anderem von der Firma New England Biolabs (Frankfurt, Deutschland) vertrieben wird oder die Pfu-Polymerase aus Pyrococcus furiosus, die unter anderem von der Firma Stratagene (La Jolla, USA) vertrieben wird. Bevorzugt werden Polymerasen mit "proof-reading"-Aktivität. "proof-reading"-Aktivität bedeutet, dass diese Polymerasen in der Lage sind, fehlerhaft eingebaute Nukleotide zu erkennen und den Fehler durch erneute Polymerisation zu beheben (Lottspeich und Zorbas, Bioanalytik, Spektrum Akademischer Verlag, Heidelberg, Deutschland (1998)). Beispiele für Polymerasen mit "proof-reading"-Aktivität sind die Vent-Polymerase und die Pfu-Polymerase.

Die Bedingungen im Reaktionsansatz werden nach Angaben des Herstellers eingestellt. Die Polymerasen werden vom Hersteller im Allgemeinen zusammen mit dem gebräuchlichen Puffer geliefert, welcher üblicherweise Konzentrationen von 10 - 100 mM Tris/HCl und 6 - 55 mM KCl bei pH 7,5 - 9,3 aufweist. Magnesiumchlorid wird in einer Konzentration von 0,5 - 10 mM zugesetzt, falls es nicht in dem vom Hersteller gelieferten Puffer enthalten ist. Dem Reaktionsansatz werden weiterhin Desoxynucleosidtriphosphate in einer Konzentration von 0,1 - 16,6 mM zugesetzt. Die Primer werden im Reaktionsansatz mit einer Endkonzentration von 0,1 - 3 µM vorgelegt und die Template-DNA im optimalen Fall mit 10² bis 10⁵ Kopien. Es können auch 10⁶ bis 10⁷ Kopien eingesetzt werden. Die entsprechende Polymerase wird dem Reaktionsansatz in einer Menge von 2-5 Units zugegeben. Ein typischer Reaktionsansatz hat ein Volumen von 20 - 100 µl.

Als weitere Zusätze können der Reaktion Rinderserumalbumin, Tween-20, Gelatine, Glycerin, Formamid oder DMSO beigesetzt werden (Dieffenbach und Dveksler, PCR Primer - A Laboratory Manual, Cold Spring Harbor Laboratory Press, USA 1995).

Ein typischer PCR-Verlauf besteht aus drei unterschiedlichen, sich aufeinanderfolgend wiederholenden Temperaturstufen. Vorab wird die Reaktion mit einer Temperaturerhöhung auf 92°C - 98°C für 4 bis 10 Minuten gestartet, um die vorgelegte DNA zu denaturieren. Dann folgen sich wiederholend zuerst ein Schritt zur Denaturierung der vorgelegten DNA von 10 - 60 Sekunden bei ungefähr 92-98°C, dann ein Schritt zum Binden der Primer an die vorgelegte DNA von 10 - 60 Sekunden bei einer bestimmten, von den Primern abhängigen Temperatur ("Annealing-Temperatur"), die erfahrungsgemäß bei 50°C bis 60°C liegt und sich für jedes Primerpaar einzeln berechnen läßt. Genaue Informationen dazu findet der Fachmann bei Rychlik et al. (Nucleic Acids Research 18 (21): 6409-6412). Anschließend folgt ein Synthese-Schritt zur Verlängerung der vorgelegten Primer ("Extension") bei dem jeweils für die Polymerase angegebenen Aktivitätsoptimum, üblicherweise je nach Polymerase im Bereich von 73°C bis 67°C bevorzugt 72°C bis 68°C. Die Dauer dieses Extensionschrittes hängt von der Leistungsfähigkeit der Polymerase und Länge des zu amplifizierenden PCR-Produktes ab. In einer typischen PCR dauert dieser Schritt 0,5 - 8 Minuten, bevorzugt 2 - 4 Minuten. Diese drei Schritte werden 30 bis 35 mal, gegebenenfalls bis zu 50 mal wiederholt. Ein abschließender "Extension"-Schritt von 4 - 10 Minuten beendet die Reaktion. Die auf diese Weise hergestellten Polynukleotide werden auch als Amplifikate bezeichnet; der Begriff Nukleinsäurefragment ist ebenfalls gebräuchlich.

Weitere Anleitungen und Informationen zur PCR findet der Fachmann beispielsweise im Handbuch "PCR-Strategies" (Innis, Felfand und Sninsky, Academic Press , Inc., 1995), im Handbuch von Diefenbach und Dveksler "PCR Primer - a laboratory manual" (Cold Spring Harbor Laboratory Press,1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994).

Die Nukleotidsequenz wird anschließend beispielsweise nach dem Kettenabbruchverfahren von Sanger et al. (Proceedings of the National Academies of Sciences, U.S.A., 74, 5463-5467 (1977)) mit den von Zimmermann et al. (Nucleic Acids Research 18, 1067pp (1990)) angegebenen Modifikationen bestimmt und das von dieser Nukleotidsequenz kodierte Polypeptid insbesondere bezüglich der Aminosäuresequenz analysiert. Dazu wird die Nukleotidsequenz in ein Programm zur Übersetzung von DNA-Sequenz in eine Aminosäuresequenz eingegeben. Geeignete Programme sind beispielsweise das Programm "Patentin", das bei Patentämtern, beispielsweise dem US-Patentamt (USPTO) erhältlich ist, oder das "Translate Tool", das auf dem ExPASy Proteomics Server im World Wide Web (Gasteiger et al., Nucleic Acids Research 31, 3784-3788 (2003)) verfügbar ist.

Auf diese Weise werden Mutanten identifiziert, deren oxyR-Allele für Polypeptide mit OxyR-Transkriptionsregulator Enzymaktivität kodieren, welche an Position 89 der Aminosäuresequenz, bzw. der entsprechenden oder vergleichbaren Position, anstelle von L-Alanin L-Valin enthalten.

Gegebenenfalls wird das gesamte Chromosom der Mutante bestimmt. Hierbei kann die von Margulies et al. (Nature, 437(7057): 376-380 (2005)) und Velicer et al. (Proceedings of the National Academy of Sciences, U.S.A., 103(21), 8107-8112 (2006)) beschriebene Methode, die unter dem Stichwort "pyro-sequencing" in der Fachwelt bekannt ist und eine rasche Sequenzierung kompletter Genome ermöglicht, eingesetzt werden.

Die auf diese Weise erzeugten Mutanten enthalten typischerweise eine (1) Kopie des beschriebenen oxyR-Allels.

In der SEQ ID NO:5 ist beispielhaft die Kodierregion des oxyR-Allels einer erfindungsgemäßen Mutante wiedergegeben. Die Kodierregion des Wildtypgens ist als SEQ ID NO:1 wiedergegeben. SEQ ID NO:1 enthält an Position 265 die Nukleobase Guanin, an Position 266 die Nukleobase Cytosin und an Position 267 die Nukleobase Cytosin. SEQ ID NO:1 enthält somit von Position 265 bis 267 das für die Aminosäure L-Alanin kodierende GCC Kodon. SEQ ID NO:5 enthält an Position 266 die Nukleobase Thymin. Durch diese Cytosin zu Thymin Transition entsteht an Position 265 bis 267 das für die Aminosäure L-Valin kodierende GTC Kodon.

Darüber hinaus können die in SEQ ID NO: 5 bzw. 7 oder SEQ ID NO:21 dargestellten Nukleotidsequenzen weitere Basenaustausche enthalten, die sich durch die Mutagenesebehandlung ergeben haben, sich jedoch nicht in einer veränderten Aminosäuresequenz äußern. Derartige Mutationen werden in der Fachwelt auch als stille oder neutrale Mutationen bezeichnet. Diese stillen Mutationen können ebenfalls in dem für die Mutagenesebehandlung eingesetzten coryneformen Bakterium bereits enthalten sein.

Die für die Mutagenese verwendeten coryneformen Bakterien besitzen bevorzugt bereits die Fähigkeit, L-Lysin in das/die sie umgebende Nährmedium bzw. Fermentationsbrühe auszuscheiden oder im Zellinneren anzureichern.

L-Lysin produzierende coryneforme Bakterien besitzen typischerweise eine "feed back" resistente bzw. desensibilisierte Aspartatkinase. Unter "feed back" resistenten Aspartatkinasen versteht man Aspartatkinasen (LysC), die im Vergleich zur Wildform eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese desensibilisierten Aspartatkinasen kodierenden Gene bzw. Allele werden auch als lysC^{FBR}-Allele bezeichnet. Im Stand der Technik sind zahlreiche lysC^{FBR}-Allele beschrieben, die für Aspartatkinasevarianten kodieren, welche Aminosäureaustausche im Vergleich zum Wildtypprotein besitzen. Die Kodierregion des Wildtyp lysC-Gens von Corynebacterium glutamicum entsprechend der Zugangsnummer AX756575 der NCBI Datenbank ist in SEQ ID NO:11 und das von diesem Gen kodierte Polypeptid in SEQ ID NO:12 dargestellt.

Die für die Maßnahmen der Erfindung eingesetzten L-Lysin produzierenden coryneformen Bakterien verfügen bevorzugt über ein lysC-Allel, das für eine Aspartatkinasevariante kodiert, welche die Aminosäuresequenz von SEQ ID NO:12 besitzt, wobei diese eine oder mehrere der Aminosäureaustausche, beispielsweise
LysC A279T (Austausch von L-Alanin an Position 279 des kodierten Aspartatkinaseproteins gemäß SEQ ID NO: 12 gegen L-Threonin; siehe US 5,688,671 und Zugangsnummern E06825, E06826, E08178 und 174588 bis I74597),
LysC A279V (Austausch von L-Alanin an Position 279 des kodierten Aspartatkinaseproteins gemäß SEQ ID NO: 12 gegen L-Valin; siehe JP 6-261766 und Zugangsnummer E08179),
LysC L297Q (Austausch von L-Leucin an Position 297 des kodierten Aspartatkinaseproteins gemäß SEQ ID NO: 12 gegen L-Glutamin; siehe DE 102006026328,
LysC S301F (Austausch von L-Serin an Position 301 des kodierten Aspartatkinaseproteins gemäß SEQ ID NO: 12 gegen L-Phenylalanin; siehe US 6,844,176 und Zugangsnummer E08180),
LysC S301Y (Austausch von L-Serin an Position 301 des kodierten Aspartatkinaseproteins gemäß SEQ ID NO: 12 gegen L-Tyrosin; siehe Kalinowski et al. (Molecular and General Genetics 224, 317-324 (1990)) und Zugangsnummer X57226),
LysC T308I (Austausch von L-Threonin an Position 308 des kodierten Aspartatkinaseproteins gemäß SEQ ID NO: 12 gegen L-Isoleucin; siehe JP 6-261766 und Zugangsnummer E08181)
LysC T311I (Austausch von L-Threonin an Position 311 des kodierten Aspartatkinaseproteins gemäß SEQ ID NO: 12 gegen L-Isoleucin; siehe WO 00/63388 und US 6,893,848),
LysC S317A (Austausch von L-Serin an Position 317 des kodierten Aspartatkinaseproteins gemäß SEQ ID NO: 12 gegen L-Alanin; siehe US 5,688,671 und Zugangsnummer I74589),
LysC R320G (Austausch von L-Arginin an Position 320 des kodierten Aspartatkinaseproteins gemäß SEQ ID NO: 12 gegen Glycin; siehe Jetten et al. (Applied Microbiology and Biotechnology 43, 76-82 (995)) und Zugangsnummer L27125),
LysC G345D (Austausch von Glycin an Position 345 des kodierten Aspartatkinaseproteins gemäß SEQ ID NO: 12 gegen L-Asparaginsäure; siehe Jetten et al. (Applied Microbiology and Biotechnology 43, 76-82 (995)) und Zugangsnummer L16848),
LysC T380I (Austausch von L-Threonin an Position 380 des kodierten Aspartatkinaseproteins gemäß SEQ ID 12: 10 gegen L-Isoleucin; siehe WO 01/49854 und Zugangsnummer AX192358), und
LysC S381F (Austausch von L-Serin an Position 381 des kodierten Aspartatkinaseproteins gemäß SEQ ID NO: 12 gegen L-Phenylalanin; siehe EP 0435132)

### umfassen kann.

Besonders bevorzugt werden das lysC^{FBR}-Allel lysC T311I (Austausch von Threonin an Position 311 des kodierten Aspartatkinaseproteins gemäß SEQ ID NO: 12 gegen Isoleucin) und ein lysC^{FBR}-Allel, enthaltend mindestens einen Austausch ausgewählt aus der Gruppe A279T (Austausch von Alanin an Position 279 des kodierten Aspartatkinaseproteins gemäß SEQ ID NO: 12 gegen Threonin), S381F (Austausch von Serin an Position 891 des kodierten Aspartatkinaseproteins gemäß SEQ ID NO: 12 gegen Phenylalanin) und S317A (Austausch von Serin an Position 317 des kodierten Aspartatkinaseproteins gemäß SEQ ID NO: 12 gegen Alanin).

Ganz besonders bevorzugt wird das lysC^{FBR}-Allel lysC T311I (Austausch von Threonin an Position 311 des kodierten Aspartatkinaseproteins gemäß SEQ ID NO: 12 gegen Isoleucin).

Der Stamm DSM 16833 (WO 06/063660) besitzt ein lysC^{FBR}-Allel, das für ein Aspartatkinaseprotein kodiert, welches den Aminosäureaustausch T311I enthält.

Der Stamm NRRL B-11474 (US 4,275,157) besitzt ein lysC^{FBR}-Allel, das für ein Aspartatkinaseprotein kodiert, welches die Aminosäureaustausche A279T und S381F enthält.

Nach der oben beschriebenen Art und Weise wurde, ausgehend von Stamm DSM 16833, eine als DM1914 bezeichnete Mutante isoliert, die ein oxyR-Allel enthält, das für ein Polypeptid kodiert, bei dem an Position 89 der Aminosäuresequenz L-Valin enthalten ist. Die Nukleotidsequenz der Kodierregion des oxyR-Allels der Mutante DM1914 ist als SEQ ID NO:5 und die Aminosäuresequenz des kodierten Polypeptids als SEQ ID NO:6 bzw. 8 dargestellt.

Weiterhin wurde nach der oben beschriebenen Art und Weise eine Mutante isoliert, die an Position 89 von SEQ ID NO:2 L-Valin anstelle L-Alanin und außerdem an Position 244 von SEQ ID NO:2 L-Valin anstelle L-Alanin enthält. Die Nukleotidsequenz des oxyR-Allels dieser Mutante ist als SEQ ID NO:21 und die Aminosäuresequenz des kodierten Polypeptids als SEQ ID NO:22 dargestellt.

Darüberhinaus können L-Lysin ausscheidende coryneforme Bakterien verwendet werden, die Eigenschaften besitzen, wie sie aus dem Stand der Technik bekannt sind.

Weiterhin können für die Mutagenese des oxyR-Gens in-vitro Methoden eingesetzt werden. Bei der Verwendung von in-vitro Methoden werden isolierte Polynukleotide, welche ein oxyR-Gen eines coryneformen Bakteriums, vorzugsweise das im Stand der Technik beschriebene Wildtyp-Gen von Corynebacterium glutamicum, enthalten, einer mutagenen Behandlung unterzogen.

Bei den isolierten Polynukleotiden kann es sich beispielsweise um isolierte Gesamt-DNA bzw. chromosomale DNA oder auch um Amplifikate des oxyR-Gens handeln, die mit Hilfe der Polymerasekettenreaktion (PCR) hergestellt wurden. Derartige Amplifikate werden auch als PCR-Produkte bezeichnet. Anleitungen zur Amplifikation von DNA-Sequenzen mit Hilfe der Polymerase-Kettenreaktion findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994). Es ist ebenfalls möglich, das zu mutagenisierende oxyR-Gen zunächst in einen Vektor, beispielsweise in einen Bakteriophagen oder in ein Plasmid einzubauen.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and OxyRated Bacteria, Cold Spring Harbor Laboratory Press, Cold Spring Harbor, 1992), die Verwendung mutagener Oligonukleotide (T. A. Brown: Gentechnologie für Einsteiger, Spektrum Akademischer Verlag, Heidelberg, 1993 und R. M. Horton: PCR-Mediated Recombination and Mutagenesis, Molecular Biotechnology 3, 93-99 (1995)) und der Einsatz einer Polymerasekettenreaktion unter Verwendung einer DNA-Polymerase, die eine hohe Fehlerquote aufweist. Eine derartige DNA-Polymerase ist beispielsweise die Mutazyme DNA Polymerase (GeneMorph PCR Mutagenesis Kit, Nr.600550) der Firma Stratagene (LaJolla, CA, USA).

Weitere Anleitungen und Übersichten zur Erzeugung von Mutationen in-vivo oder in-vitro können dem Stand der Technik und bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Gegenstand der Erfindung ist weiterhin ein isoliertes Polynukleotid, dass für ein Polypeptid mit OxyR-Transkriptionsregulator-Aktivität kodiert, welches die Aminosäuresequenz von SEQ ID NO:6 umfasst oder die Aminosäuresequenz gemäß SEQ ID NO: 6 umfasst, die zusätzlich maximal fünf konservative Aminosäureaustausche und maximal 5 Insertionen oder Deletionen enthält, wobei die maximal fünf konservativen Aminosäureaustausche und maximal 5 Insertionen die Aktivität im Wesentlichen nicht verändern. (vgl. ursprünglicher Anspruch 27; S. 36, Z. 32-S. 37, Z.6; S. 37, Z. 16-22)

Die Aminosäuresequenz gemäß SEQ ID NO: 6 ist dadurch entstanden, dass an Position 89 der Aminosäuresequenz gemäß SEQ ID NO: 2 L-Alanin gegen L-Valin ausgetauscht ist.

Das erfindungsgemäße, isolierte Polynukleotid kodiert vorzugsweise für ein Polypeptid mit OxyR-Transkriptionsregulator-Aktivität, welches eine Aminosäuresequenz mit einer Länge von 327 Aminoäuren umfasst.

Gegenstand der Erfindung ist weiterhin ein isoliertes Polynukleotid, das identisch ist mit der Nukleotidsequenz von SEQ ID NO:5, wobei gegebenenfalls an Position 731 Thymin anstelle von Cytosin enthalten ist.

Gegenstand der Erfindung ist weiterhin ein isoliertes Polynukleotid, das die Nukleotidsequenz gemäß SEQ ID NO:5 oder SEQ ID NO:7 umfasst.

Gegenstand der Erfindung ist außerdem ein isoliertes Polynukleotid, das einen Teil der Kodierregion eines erfindungsgemäßen oxyR-Allels umfasst, wobei das isolierte Polynukleotid in jedem Fall den Teil der Kodierregion umfasst, der den Aminosäureaustausch an der Position 89 der Aminosäuresequenz des kodierten Polypeptids enthält und das für mindestens eine Aminosäuresequenz entsprechend Position 70 bis 108 von SEQ ID NO:2 kodiert, wobei an der Position entsprechend 89 von SEQ ID NO:2 anstelle von L-Alanin L-Valin, enthalten ist und wobei gegebenenfalls an Position 244 L-Valin enthalten ist.

Bevorzugt werden Nukleinsäuremoleküle die für mindestens eine Aminosäuresequenz entsprechend Position 70 bis 108 von SEQ ID NO:6.

Ein Beispiel eines erfindungsgemäßen Leserasters umfassend ein Polynukleotid, das für mindestens die Aminosäuresequenz entsprechend Position 70 bis 108 von SEQ ID NO:6 kodiert, ist nachfolgend aufgeführt:

Das Leseraster ist ebenfalls als SEQ ID NO:19 dargestellt. SEQ ID NO:20 zeigt die von diesem Leseraster kodierte Aminosäuresequenz. Die Position 20 in SEQ ID NO:20 entspricht der Position 89 von SEQ ID NO:2, 4, 6 bzw. 8.

Ganz besonders bevorzugt werden Nukleinsäuremoleküle, die mindestens eine Nukleotidsequenz entsprechend Position 958 bis 1074 von SEQ ID NO:7 umfassen, wobei gegebenenfalls an Position 731 Thymin enthalten ist.

Die erfindungsgemäßen Leseraster, wie sie beispielhaft in SEQ ID NO: 19 als Nukleotidsequenz und in SEQ ID NO:20 in Form der kodierten Aminosäuresequenz gezeigt sind, können darüberhinaus eine oder mehrere Mutationen enthalten, die zu einem oder mehreren konservativen Aminosäureaustauschen führen.

Die erfindungsgemäßen, isolierten Polynukleotide können dazu verwendet werden, um rekombinante Stämme von Mikroorganismen herzustellen, die im Vergleich zum Ausgangs- bzw. Elternstamm in verbesserter Weise Aminosäuren in das sie umgebende Medium abgeben oder im Zellinneren anhäufen.

Eine verbreitete Methode zum Einbau von Mutationen in Gene coryneformer Bakterien ist die des Allelaustausches, die auch unter der Bezeichnung "gene replacement" bekannt ist. Bei diesem Verfahren wird ein DNA-Fragment, welches die interessierende Mutation enthält, in den gewünschten Stamm eines coryneformen Bakteriums transferiert und die Mutation durch wenigstens zwei Rekombinationsereignisse bzw. "cross over"-Ereignisse in das Chromosom des gewünschten Stammes inkorporiert bzw. die im betreffenden Stamm vorhandene Sequenz eines Gens gegen die mutierte Sequenz ausgetauscht.

Von Schwarzer und Pühler (Bio/Technology 9, 84-87 (1991)) wurde diese Methode verwendet um ein lysA-Allel, welches eine Deletion trug, und ein lysA-Allel, welches eine Insertion trug, in das Chromosom von C. glutamicum anstelle des Wildtypgens einzubauen. Von Schäfer et al. (Gene 145, 69-73 (1994)) wurde diese Methode eingesetzt, um eine Deletion in das hom-thrB Operon von C. glutamicum einzubauen. Von Nakagawa et al. (EP 1108790) und Ohnishi et al. (Applied Microbiology and Biotechnology 58(2), 217-223 (2002)) wurde diese Methode eingesetzt, um verschiedene Mutationen ausgehend von den isolierten Allelen in das Chromosom von C. glutamicum einzubauen. Auf diese Weise gelang es Nakagawa et al. eine als Val59Ala bezeichnete Mutation in das Homoserin-Dehydrogenase-Gen (hom), eine als Thr311Ile bezeichnete Mutation in das Aspartatkinase-Gen (lysC bzw. ask), eine als Pro458Ser bezeichnete Mutation in das Pyruvat-Carboxylase-Gen (pyc) und eine als Ala213Thr bezeichnete Mutation in das Glucose-6-phosphat-Dehydrogenase-Gen (zwf) von C. glutamicum Stämmen einzubauen.

Für ein erfindungsgemäßes Verfahren kann ein erfindungsgemäßes Polynukleotid verwendet werden, welches die gesamte Kodierregion umfasst, wie beispielsweise in SEQ ID NO:5 gezeigt, oder welches einen Teil der Kodierregion umfasst, wie beispielsweise die Nukleotidsequenz, die für mindestens die Aminosäuresequenz entsprechend Position 70 bis 108 von SEQ ID NO:6 kodiert und die als SEQ ID NO: 19 dargestellt ist. Der Teil der Kodierregion entsprechend SEQ ID NO: 19 umfasst eine Länge von ≥ 117 Nukleobasen. Bevorzugt werden solche Teile von SEQ ID NO:7, die mindestens die Sequenz zwischen Position 1194 und 838 aufweisen und dementsprechend eine Länge von ≥ 357 Nukleobasen besitzen. Besonders bevorzugt werden solche Teile von SEQ ID NO:7, die mindestens die Sequenz zwischen Position 1314 und 718 aufweisen und dementsprechend eine Länge von ≥ 597 Nukleobasen besitzen. Ganz besonders bevorzugt werden solche Teile von SEQ ID NO:7, die mindestens die Sequenz zwischen Position 1434 und 598 aufweisen und dementsprechend eine Länge von ≥ 837 Nukleobasen besitzen.

Das DNA-Fragment enthaltend die interessierende Mutation liegt bei dieser Methode typischerweise in einem Vektor, insbesondere einem Plasmid, vor, das vorzugsweise von dem mit der Mutation zu versehenden Stamm nicht oder nur begrenzt repliziert wird. Als Hilfs- oder Zwischenwirt, in dem der Vektor replizierbar ist, wird im Allgemeinen ein Bakterium der Gattung Escherichia bevorzugt der Spezies Escherichia coli verwendet.

Beispiele für derartige Plasmidvektoren sind die von Schäfer et al. (Gene 145, 69-73 (1994)) beschriebenen pK*mob und pK*mobsacB Vektoren, wie beispielsweise pK18mobsacB, und die in der WO 02/070685 und WO 03/014362 nicht in coryneformen Bakterien replikativ. Besonders geeignet sind Vektoren, die ein konditional negativ dominant wirkendes Gen wie beispielsweise das sacB-Gen (Levansucrase-Gen) von beispielsweise Bacillus oder das galK-Gen (Galaktosekinase-Gen) von beispielsweise Escherichia coli enthalten. (Unter einem konditional negativ dominant wirkenden Gen versteht man ein Gen, das unter bestimmten Bedingungen nachteilig beispielsweise toxisch für den Wirt ist, unter anderen Bedingungen aber keine negativen Auswirkungen auf den das Gen tragenden Wirt hat.) Diese ermöglichen die Selektion auf Rekombinationsereignisse, bei denen der Vektor aus dem Chromosom eliminiert wird. Weiterhin wurde von Nakamura et al. (US-A-6,303,383) ein Temperatur-sensitives Plasmid für coryneforme Bakterien beschrieben, das lediglich bei Temperaturen unterhalb von 31°C replizieren kann.

Der Vektor wird anschließend durch Konjugation beispielsweise nach der Methode von Schäfer (Journal of Bacteriology 172, 1663-1666 (1990)) oder Transformation beispielsweise nach der Methode von Dunican und Shivnan (Bio/Technology 7, 1067-1070 (1989)) oder der Methode von Thierbach et al. (Applied Microbiology and Biotechnology 29, 356-362 (1988)) in das coryneforme Bakterium transferiert. Gegebenenfalls kann der Transfer der DNA auch durch Partikelbeschuss erzielt werden.

Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation und erhält ein rekombinantes Bakterium.

Zur Identifizierung und Charakterisierung der erhaltenen Stämme können unter anderem die Methoden der Southern Blotting Hybridisierung, der Polymerase-Kettenreaktion, der Sequenzbestimmung, die Methode des "Fluorescence Resonance Energy Transfer" (FRET) (Lay et al. Clinical Chemistry 43, 2262-2267 (1997)) oder Methoden der Enzymologie eingesetzt werden.

Auf diese Weise erhält man einen rekombinanten Mikroorganismus, welcher mindestens eine (1) Kopie oder mehrere Kopien eines erfindungsgemäßen Polynukleotids enthält, das für einen OxyR-Transkriptionsregulator kodiert, der an Position 89 L-Valin enthält.

Weitere Gegenstände der Erfindung sind dementsprechend Wirte bzw. Wirtszellen, bevorzugt Mikroorganismen, besonders bevorzugt coryneforme Bakterien und Bakterien der Gattung Escherichia, die die erfindungsgemäßen Polynukleotide enthalten. Gegenstand der Erfindung sind gleichfalls Mikrorganismen, die unter Verwendung der isolierten Polynukleotide hergestellt wurden. Derartige Mikroorganismen oder Bakterien werden auch als rekombinante Mikroorganismen oder rekombinante Bakterien bezeichnet. In gleicher Weise sind Vektoren, die die erfindungsgemäßen Polynukleotide enthalten, Gegenstand der Erfindung. Schließlich sind Wirte, die diese Vektoren enthalten, ebenfalls Gegenstand der Erfindung.

Zusätzlich kann es für die erhöhte Produktion von L-Lysin vorteilhaft sein, in den erfindungsgemäßen Mutanten oder rekombinanten Stämmen verschiedene Gene zu überexprimieren. Die Verwendung endogener Gene wird im Allgemeinen bevorzugt.

Unter "endogenen Genen" oder "endogenen Nukleotidsequenzen" versteht man die in der Population einer Art vorhandenen Gene bzw. Nukleotidsequenzen oder Allele.

So kann für die Herstellung von L-Lysin eines oder mehrere der Gene, ausgewählt aus der Gruppe
- ein für ein Dihydrodipicolinat-Synthase (DapA, EC Nr. 4.2.1.52) kodierendes Gen dapA, wie beispielsweise das in der EP 0 197 335 beschriebene dapA-Gen des Wildtyps von Corynebacterium glutamicum,
- ein für eine Diaminopimelat-Decarboxylase (LysA, EC Nr. 4.1.1.20) kodierendes lysA-Gen, wie beispielsweise das in in der US 6,090,597 beschriebene lysA-Gen von Corynebacterium glutamicum ATCC13869,
- ein für eine Glucose-6-Phosphat Dehydrogenase (Zwf, EC Nr. 1.1.1.49) kodierendes Gen zwf, wie beispielsweise das in der JP-A-09224661 und EP-A-1108790 beschriebene zwf-Gen des Wildtyps von Corynebacterium glutamicum,
- die in der US-2003-0175911-A1 beschriebenen zwf-Allele von Corynebacterium glutamicum, die für ein Protein mit Glucose-6-Phosphat Dehydrogenase Aktivität kodieren, bei dem beispielsweise das L-Alanin an Position 243 der Aminosäuresequenz durch L-Threonin ersetzt ist oder bei dem die L-Asparaginsäure an Position 245 durch L-Serin ersetzt ist, oder bei dem die L-Asparaginsäure an Postion 245 durch L-Serin ersetzt ist,
- ein für eine Pyruvat-Carboxylase (Pyc, EC Nr. 6.4.1.1) kodierendes Gen pyc, wie beispielsweise das in der DE-A-198 31 609 und EP 1108790 beschriebene pyc-Gen des Wildtyps von Corynebacterium glutamicum,
- das in der EP 1 108 790 beschriebene pyc-Allel von Corynebacterium glutamicum, das für ein Protein mit Pyruvat-Carboxylase Aktivität kodiert, bei dem L-Prolin an Position 458 der Aminosäuresequenz durch L-Serin ersetzt ist,
- die in der WO 02/31158 und insbesondere EP1325135B1 beschriebene pyc-Allele von Corynebacterium glutamicum, die für Proteine mit Pyruvat-Carboxylase Aktivität kodieren, welche einen oder mehrere der Aminosäureaustausche ausgewählt aus der Gruppe L-Valin an Position 1 ersetzt durch L-Methionin, L-Glutaminsäure an Position 153 ersetzt durch L-Asparaginsäure, L-Alanin an Position 182 ersetzt durch L-Serin, L-Alanin an Position 206 ersetzt durch L-Serin, L-Histidin an Position 227 ersetzt durch L-Arginin, L-Alanin an Position 455 ersetzt durch Glycin und L-Asparaginsäure an Position 1120 ersetzt durch L-Glutaminsäure tragen
- ein für eine Aspartatkinase (LysC, EC Nr. 2.7.2.4) kodierendes lysC-Gen wie beispielsweise das als SEQ ID NO:281 in der EP-A-1108790 (Siehe auch Zugangsnummer AX120085 und 120365) und das als SEQ ID NO:25 in der WO 01/00843 (Siehe Zugangsnummer AX063743) beschriebene von lysC-Gen des Wildtyps von Corynebacterium glutamicum,
- ein für eine feed-back resistente Aspartatkinase Variante kodierendes lysC^{FBR} Allel, insbesondere entsprechend Tabelle 1,
- ein für ein Lysin-Export-Protein (LysE) kodierendes Gen lysE, wie beispielsweise das in der DE-A-195 48 222 beschriebene lysE-Gen von des Wildtyps Corynebacterium glutamicum,
- das für eine Aspartat-Aminotransferase (Aat, EC Nr. 2.6.1.1) kodierende aat-Gen (Das aat-Gen von Corynebacterium glutamicum ATCC13032 ist beispielsweise bei Kalinowski et al. (Journal of Biotechnology 104 (1-3), 5-25 (2003); siehe auch Zugangsnummer NC_006958) beschrieben. Es wird dort als aspB-Gen bezeichnet. In der US 6,004,773 wird ein für eine Aspartat-Aminotransferase kodierendes Gen als aspC bezeichnet. Marienhagen et al (Journal of Bacteriology 187 (22), 7693-7646 (2005) bezeichnen das aat-Gen als aspT-Gen.)),
- das für das Zwa1-Protein kodierende Gen zwa1 des Wildtyps von Corynebacterium glutamicum (US 6,632,644)
überexprimiert werden.

Weiterhin kann es für die Produktion von L-Lysin vorteilhaft sein, neben der Verwendung der erfindungsgemäßen Allele des oxyR-Gens gleichzeitig, gegebenenfalls bei gleichzeitiger Überexpression von mindestens einem der Gene ausgewählt aus der vorstehend genannten Gruppe von Genen, eines oder mehrere der endogenen Gene, ausgewählt aus der Gruppe
• ein für die Glucose-6-Phosphat-Isomerase (Pgi, EC Nr. 5.3.1.9) kodierendes Gen pgi, wie beispielsweise das in der US 6,586,214 und US 6,465,238 beschriebene pgi-Gen von Corynebacterium glutamicum,
• ein für die Homoserin-Dehydrogenase(Hom, EC Nr. 1.1.1.3) kodierendes Gen hom, wie beispielsweise das in der EP-A-0131171 beschriebene hom-Gen von Corynebacterium glutamicum,
• ein für die Homoserin-Kinase (ThrB, EC Nr. 2.7.1.39) kodierendes Gen thrB, wie beispielsweise das von Peoples et al. (Molecular Microbiology 2 (1988): 63 - 72) beschriebene thrB-Gen von Corynebacterium glutamicum und
• ein für die Phosphofruktokinase(PfkB, EC Nr. 2.7.1.56) kodierendes Gen pfkB, wie beispielsweise das in der WO 01/00844 (Sequenz Nr. 57) beschriebene pfkB-Gen von Corynebacterium glutamicum,
• ein für die Malat-Dehydrogenase (Mdh, EC Nr. 1.1.1.37) kodierendes Gen mdh, wie beispielsweise in der WO 02/02778 beschrieben,
• ein für die Malat-Chinon Oxidoreduktase (Mqo, EC Nr. 1.1.99.16) kodierendes Gen mqo, wie beispielsweise in der US 7,094,106 und PCT/EP2005/057216 beschrieben abzuschwächen oder auszuschalten.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im Allgemeinen auf 0 bis 75%, 0 bis 50%, 0 bis 25%, 0 bis 10% oder 0 bis 5% der Aktivität oder Konzentration des Wildtyp-Proteins, bzw. der Aktivität oder Konzentration des Proteins im Ausgangs-Mikroorganismus, herabgesenkt.

Als Mutationen zur Erzeugung einer Abschwächung kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nichtkonservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stopp-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stopp-Kodon im Kodierbereich, so führt dies ebenfalls zu einem vorzeitigen Abbruch der Translation. Die genannten Maßnahmen werden vorzugsweise im 5'-terminalen Teil der Kodierregion durchgeführt, welcher für den N-Terminus des Polypeptids kodiert. Bezeichnet man die Gesamtlänge eines Polypeptids (gemessen als Anzahl der chemisch verbundenen L-Aminosäuren) mit 100%, so gehört - im Rahmen der vorliegenden Erfindung - zum N-Terminus des Polypeptids der Teil der Aminosäuresequenz, welcher, gerechnet ab der Start-Aminosäure L-Formyl-Methionin 80% der nachfolgenden L-Aminosäuren enthält.

Weitere Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden. Weitere Maßnahmen sind im Stand der Technik beschrieben.

Ein Methode zur gezielten Verringerung der Genexpression besteht darin, das abzuschwächende Gen unter die Kontrolle eines durch Zugabe dosierter Mengen von IPTG (Isopropyl-*β-*D-thiogalactopyranosid) induzierbaren Promotors, wie zum Beispiel des trc-Promotors oder des tac-Promotors, zu stellen. Hierzu eignen sich Vektoren wie beispielsweise der Escherichia coli Expressionsvektor pXK99E (WO0226787; hinterlegt gemäß Budapester Vertrag am 31. Juli 2001 in DH5alpha/pXK99E als DSM14440 bei der Deutschen Sammlung für Mikroorganismen und Zellkulturen (DSMZ, Braunschweig, Deutschland)) oder pVWEx2 (Wendisch, Ph. D. thesis, Berichte des Forschungszentrums Jülich, Jül-3397, ISSN 0994-2952, Jülich, Deutschland (1997)), die eine IPTGabhängige Expression des klonierten Gens in Corynebacterium glutamicum ermöglichen.

Eingesetzt wurde diese Methode beispielsweise in der Patentschrift WO0226787 zur regulierten Expression des deaD-Gens durch Integration des Vektors pXK99EdeaD in das Genom von Corynebacterium glutamicum und von Simic et al. (Applied and Environmental Microbiology 68: 3321-3327 (2002)) zur regulierten Expression des glyA-Gens durch Integration des Vektors pK18mobglyA' in Corynebacterium glutamicum.

Eine weitere Methode zur spezifischen Verringerung der Genexpression ist die Antisense-Technik, wobei kurze Oligodesoxynukleotide oder Vektoren zur Synthese längerer Antisense-RNA in die Zielzellen gebracht werden. Die Antisense-RNA kann dort an komplementäre Abschnitte spezifischer mRNAs binden und deren Stabilität verringern oder die Translatierbarkeit blocken. Ein Beispiel hierzu findet der Fachmann bei Srivastava et al. (Applied Environmental Microbiology 2000 Oct; 66 (10): 4366 - 4371). Die durch die Maßnahmen der Erfindung erhaltenen isolierten coryneformen Bakterien zeigen eine im Vergleich zum eingesetzten Ausgangsstamm bzw. Elternstamm erhöhte Ausscheidung bzw. Produktion der gewünschten Aminosäure in einem Fermentationsprozess.

Unter isolierten Bakterien sind die erfindungsgemäßen, isolierten bzw. erzeugten Mutanten und rekombinanten Bakterien, insbesonders coryneformer Bakterien, zu verstehen, welche ein oxyR-Allel enthalten, das für einen OxyR-Transkriptionsregulator kodiert, der den beschriebenen Aminosäureaustausch an Position 89 der Aminosäuresequenz enthält.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben verbessert.

Die erfindungsgemäßen, isolierten coryneformen Bakterien können kontinuierlich - wie beispielsweise in der PCT/EP2004/008882 beschrieben- oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium bzw. Fermentationsmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden. Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoffhaltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Geeignete Fermentationsmedien sind unter anderem in der US 6,221,636, in der US 5,840,551, in der US 5,770,409, in der US 5,605,818, in der US 5,275,940, in der US 4,275,157 und in der US 4,224,409 beschrieben.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Gegenstand der Erfindung ist dementsprechend ein Verfahren zur Herstellung von L-Lysin bei dem man
a) das erfindungsgemäße isolierte coryneforme Bakterium in einem geeignetem Medium fermentiert und
b) die L-Lysin in der Fermentationsbrühe oder in den Zellen des isolierten coryneformen Bakteriums akkumuliert.

Daran schließt sich im Allgemeinen das Sammeln (collecting) der im Nährmedium bzw. in der Fermentationsbrühe und/oder in den Zellen der Bakterien akkumulierten L-Aminosäure an, um zu einem festen oder flüssigen Produkt zu gelangen.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium bzw. die während der Fermentation eingesetzten Medien enthält/enthalten sämtliche Substanzen bzw. Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) das im Laufe der Fermentation gebildete L-Lysin, c) die im Laufe der Fermentation gebildeten organischen Nebenprodukte und d) die durch die Fermentation nicht verbrauchten Bestandteile des/der eingesetzten Fermentationsmediums/Fermentationsmedien bzw. der Einsatzstoffe wie beispielsweise Vitamine wie Biotin, Aminosäuren wie Homoserin oder Salze wie Magnesiumsulfat.

Zu den organischen Nebenprodukten gehören Stoffe, die von den bei der Fermentation eingesetzten Mikroorganismen gegebenenfalls neben der jeweiligen, erwünschten L-Aminosäure erzeugt und gegebenenfalls ausgeschieden werden. Hierzu zählen L-Aminosäuren, die im Vergleich zur erwünschten Aminosäure weniger als 30%, 20% oder 10% ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben typischerweise einen Aminosäuregehalt von 30 g/kg bis 200 g/kg oder 40 g/kg bis 175 g/kg oder 50 g/kg bis 150 g/kg. Der Gehalt an Biomasse (als getrocknete Biomasse) beträgt im Allgemeinen 20 bis 50 g/kg.

Im Falle der Aminosäure L-Lysin sind im Stand der Technik im Wesentlichen vier verschiedene Produktformen bekannt.

Eine Gruppe L-Lysin-haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem bzw. reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0%-100%.

Entsprechend der verschiedenen Produktformen kennt man verschiedenste Verfahren, bei denen die L-Aminosäure aus der Fermentationsbrühe gesammelt, isoliert oder gereinigt wird, um das L-Lysin haltige Produkt oder gereinigtes L-Lysin herzustellen.

Zur Herstellung von festem, reinem L-Lysin werden im Wesentlichen Methoden der Ionenaustauschchromatographie gegebenenfalls unter Verwendung von Aktivkohle und Methoden der Kristallisierung angewendet. Man erhält auf diese Weise die entsprechende Base oder ein entsprechendes Salz wie beispielsweise das Monohydrochlorid (Lys-HCl) oder das Lysinsulfat (Lys₂-H₂SO₄).

Für Lysin ist in der EP-B-0534865 ein Verfahren zur Herstellung wässriger, basischer L-Lysin-haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden auch solche Verfahren eingesetzt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse bzw. die Biomasse enthaltene Fermentationsbrühe während eines geeigneten Verfahrensschrittes inaktiviert, beispielsweise durch thermische Behandlung (Erhitzen) oder durch Säurezugabe.

Die chemischen Bestandteile der Biomasse sind unter anderem die Zellhülle, beispielsweise das Peptidoglykan und das Arabinogalaktan, das Protein bzw. Polypeptid, beispielsweise das OxyR-Transkriptionsregulator Polypeptid, Lipide und Phospholipide und Nukleinsäuren (DNA und RNA), beispielsweise Polynukleotide enthaltend die erfindungsgemäße Mutation. Als Folge der Maßnahmen der Inaktivierung und/oder der weitereren Verfahrensschritte (beispielsweise Ansäuerung, Sprühtrocknung, Granulation etc.) liegen Nukleinsäuren typischerweise als Fragmente mit eine Länge von unter anderem ≥40 - 60 bp, >60 - 80 bp, >80 - 100 bp, >100 - 200 bp, >200 - 300 bp, >300 - 400 bp, >400 - 500 bp, >500 - 750 bp, >750 - 1000 bp, >1000 -1250 bp, >1250 - 1500 bp, >1500 - 1750 bp, >1750 - 2000 bp, >2000 - 2500 bp, >2500 - 3000 bp, >3000 - 4000 bp, >4000 - 5000 bp vor.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH-Wert gestellt werden (GB 1,439,728 oder EP 1 331 220). Es ist gleichfalls möglich die Fermentationsbrühe mit der vollständig enthaltenen Biomasse anzusäuern (US 6,340,486 oder US 6,465,025). Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig das heißt > 95% oder > 98% im Produkt. In diesem Sinne bedeutet der Begriff "Fermentationsbrühebasis", dass ein Produkt mindestens einen Teil der Bestandteile der Fermentationsbrühe enthält.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen bzw. eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Ein weiterer Gegenstand der Erfindung ist dementsprechend ein Verfahren zur Herstellung eines L-Lysin haltigen Produktes, bevorzugt Tierfuttermittel-Additivs, aus Fermentationsbrühen, gekennzeichnet durch die Schritte
a) Kultivierung und Fermentation eines erfindungsgemäßen, L-Lysin ausscheidenden coryneformen Bakteriums in einem Fermentationsmedium,
b) Entfernung der während der Fermentation gebildeten Biomasse in einer Menge von 0 bis 100 Gew.-%, und
c) Trocknung der gemäß a) und/oder b) erhaltenen Fermentationsbrühe, um das Produkt in der gewünschten Pulver- oder Granulatform zu erhalten
wobei gegebenenfalls vor Schritt b) oder c) eine Säure ausgewählt aus der Gruppe Schwefelsäure, Phosphorsäure oder Salzsäure hinzugefügt wird. Vorzugsweise wird im Anschluss an Schritt a) oder b) Wasser aus der L-Aminosäure haltigen Fermentationsbrühe entfernt (Aufkonzentration).

Ein weiterer Gegenstand der Erfindung ist ein Verfahren zur Herstellung eines Lysinsulfat haltigen Produktes, das in seinen Grundzügen in der DE 102006016158 beschrieben ist, und bei dem die unter Verwendung der erfindungsgemäßen Mikroorganismen erhaltene Fermentationsbrühe, aus der die Biomasse gegebenenfalls ganz oder teilweise abgetrennt wurde, weiterverarbeitet wird, indem man das so erhaltene Gemisch durch Wasserentzug aufkonzentriert, und gegebenenfalls granuliert.

Vorteilhaft bei der Granulation oder Kompaktierung ist der Einsatz von üblichen organischen oder anorganischen Hilfsstoffen, bzw. Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) oder Stearaten.

Weiterhin ist es vorteilhaft die Oberfläche der erhaltenen Granulate mit Ölen zu versehen so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethycellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Der Stamm MH20-22B wurde am 28. Oktober 2004 bei der Deutschen Sammlung für Mikroorganismen und Zellkulturen (DSMZ, Braunschweig, Deutschland) als DSM 16835 hinterlegt.

Die erfindungsgemäße Mutante Corynebacterium glutamicum DM1914, die L-Valin an Position 89 der Aminosäuresequenz des OxyR-Polypeptids enthält, wurde am 15. Mai 2006 bei der Deutschen Sammlung für Mikroorganismen und Zellkulturen (DSMZ, Braunschweig, Deutschland) als DSM 18259 hinterlegt.

Die vorliegende Erfindung wird im Folgenden anhand von Ausführungsbeispielen näher erläutert.

### Beispiel 1

### Mutagenese des L-Lysin-produzierenden Stammes DM1797

Der Corynebacterium glutamicum Stamm DM1797 wurde als Ausgangsstamm für die Mutagenese mit N-Methyl-N'-Nitro-N-Nitrosoguanidin (MNNG) eingesetzt. Der Stamm DM1797 ist eine Aminoethylcystein-resistente Mutante von Corynebacterium glutamicum ATCC13032 und unter der Bezeichnung DSM16833 bei der Deutschen Sammlung für Mikroorganismen und Zellkulturen (DSMZ, Braunschweig, Deutschland) hinterlegt.

Der Stamm DM1797 wurde in 10 ml LB-Bouillon (Merck, Darmstadt, Germany), die in einem 100 ml Erlenmeyerkolben enthalten waren, für 24 Stunden bei 33°C und 200 rpm auf einem Rotations-Schüttler vom Typ Certomat BS-1 (B. Braun Biotech International, Melsungen, Deutschland) kultiviert. Anschließend wurde die Kultur abzentrifugiert, das Sediment in 10 ml 0,9% NaCl-Lösung resuspendiert, die erhaltene Suspension erneut abzentrifugiert und das erhaltene Sediment in 10 ml 0,9% NaCl-Lösung aufgenommen. 5 ml dieser Zellsuspension wurden mit 400µg/ml MNNG für 15 Minuten bei 30°C und 200 rpm auf einem Schüttler (siehe oben) behandelt. Anschließend wurde der Mutageneseansatz abzentrifugiert und das Sediment in 10 ml 2% Na-Thiosulfat in 0,9% NaCl-Puffer (pH = 6,0) aufgenommen. Die Zellsuspension wurde anschließend im Verhältnis 1:1000, 1:10000 und 1:100000 mit 0,9% NaCl-Lösung verdünnt, und Aliquots auf Hirn-Herz-Agar (Merck, Darmstadt, Deutschland) ausplattiert. Auf diese Weise wurden ungefähr 2500 Mutanten isoliert.

### Beispiel 2

### Leistungstest der Mutanten des Stammes DM1797

Die in Beispiel 1 erhaltenen Mutanten wurden in einem zur Produktion von L-Lysin geeigneten Nährmedium kultiviert und der Lysingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkultur wurde das Medium MM verwendet. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet.

| Medium MM | |
|---|---|
| CSL | 5 g/l |
| MOPS | 20 g/l |
| Glucose (getrennt autoklaviert) | 50 g/l |
| Salze: | |
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug 33°C, die Umdrehungszahl 250 rpm und die Luftfeuchte 80%.

Nach 24 Stunden wurde die optische Dichte (OD) bei einer Meßwellenlänge von 660 nm mit dem Biomek 1000 (Beckmann Instruments GmbH, München, Deutschland) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt. Eine Mutante, die sich durch eine erhöhte Lysinbildung auszeichnete, wurde als DM1914 bezeichnet.

**Tabelle 1**

| Stamm | OD (660) | Lysin-HCl (g/l) |
|---|---|---|
| DM1797 | 9,2 | 2,23 |
| DM1914 | 9,2 | 2,40 |

### Beispiel 3

### Sequenzierung des oxyR-Allels der Mutante DM1914

Aus dem Klon DM1914 wurde mit der Methode von Eikmanns et al. (Microbiology 140: 1817 - 1828 (1994)) chromosomale DNA isoliert. Mit Hilfe der Polymerase-Kettenreaktion wurde ein DNA-Abschnitt amplifiziert, welcher das oxyR-Gen bzw. Allel trägt. Aufgrund der für C. glutamicum bekannten Sequenz des oxyR-Gens (Sequenz Nr. 2114 aus EP1108790) wurden folgende Primer-Oligonukleotide für die PCR ausgewählt:
oxyR_XL_A1 (SEQ ID NO: 9):
   5' gcgaattcgg gcatttacca tcatggtg 3'
oxyR_XL_A2 (SEQ ID NO: 10):
   5' gcgaattccg ctaatgcagt aggcattc 3'

Die dargestellten Primer wurden von der Firma MWG Biotech (Ebersberg, Deutschland) synthetisiert und nach der Standard-PCR-Methode von Innis et al. (PCR Protocols. A Guide to Methods and Applications, 1990, Academic Press) die PCR Reaktion durchgeführt. Die Primer ermöglichen die Amplifizierung eines ca. 1,6 kb langen DNA-Abschnittes, welcher das oxyR-Gen bzw. Allel trägt. Außerdem enthalten die Primer die Sequenz für eine Schnittstelle der Restriktionsendonuklease EcoRI, die in der oben dargestellten Nukleotidabfolge durch Unterstreichen markiert ist.

Das amplifizierte DNA-Fragment von ca. 1,6 kb Länge, welches das oxyR-Allel des Stammes DM1914 trägt, wurde durch Elektrophorese in einem 0,8%igen Agarosegel identifiziert, aus dem Gel isoliert und mit den üblichen Methoden aufgereinigt (QIAquick Gel Extraction Kit, Qiagen, Hilden).

Die Nukleotidsequenz des amplifizierten DNA-Fragmentes bzw. PCR-Produktes wurde von der Firma Agowa (Berlin, Deutschland) durch Sequenzierung ermittelt. Die Sequenz des PCR-Produktes ist in der SEQ ID NO: 15 dargestellt. Die Sequenz der Kodierregion ist zusätzlich in der SEQ ID NO: 5 dargestellt. Die sich mit Hilfe des Programmes Patentin ergebende Aminosäuresequenz des dazugehörigen OxyR-Transkriptionsregulator-Proteins ist in der SEQ ID NO: 6 dargestellt.

An der Position 266 der Nukleotidsequenz der Kodierregion des oxyR-Allels von Stamm DM1914 befindet sich die Base Thymin (SEQ ID NO: 5). An der entsprechenden Position des Wildtypgens befindet sich die Base Cytosin (SEQ ID NO: 1).

An der Position 89 der Aminosäuresequenz des OxyR-Transkriptionsregulator-Proteins von Stamm DM1914 befindet sich die Aminosäure Valin (SEQ ID NO: 6). An der entsprechenden Position des Wildtyp-Proteins befindet sich die Aminosäure Alanin (SEQ ID NO: 2).

Das oxyR-Allel, welches an der Position 266 der Kodierregion die Base Thymin enthält und dementsprechend für ein OxyR-Transkriptionsregulator-Protein kodiert, welches an Position 89 der Aminosäuresequenz die Aminosäure Valin enthält, wird im Folgenden als oxyR_A89V-Allel bezeichnet. Bei der Bezeichnung "oxyR_A89V" steht A für L-Alanin, V für L-Valin und 89 gibt die Position des Aminosäureaustausches an (Siehe SEQ ID NO: 2 und 6).

Die Corynebacterium glutamicum Mutante DM1914, die L-Valin an Position 89 der Aminosäuresequenz des OxyR-Polypeptids enthält, wurde am 15. Mai 2006 bei der Deutschen Sammlung für Mikroorganismen und Zellkulturen (DSMZ, Braunschweig, Deutschland) als DSM 18259 hinterlegt.

### Beispiel 4

Austausch des oxyR-Wildtypgens von Stamm DM1797 gegen das oxyR_A89V-Allel

### 4.1 Konstruktion des Austauschvektors pK18mobsacB_oxyR_A89V

Das in Beispiel 3 beschriebene, mittels PCR hergestellte DNA-Fragment von ca. 1,6 kb Länge, welches das oxyR_A89V-Allel trägt, wurde mittels Austauschmutagenese unter Zuhilfenahme des bei Schäfer et al. (Gene, 14, 69-73 (1994)) beschriebenen sacB-Systems in das Chromosom des in Beispiel 1 beschriebenen C. glutamicum Stammes DM1797 eingebaut. Dieses System ermöglicht die Herstellung bzw. die Selektion von Allel-Austauschen, die sich durch homologe Rekombination vollziehen.

Dazu wurde das ca. 1,6 kb große oxyR_A89V-Fragment mit der Restriktionsendonuklease EcoRI gespalten, durch Elektrophorese in einem 0,8%igen Agarosegel identifiziert und anschließend aus dem Gel isoliert und mit den üblichen Methoden aufgereinigt (QIAquick Gel Extraction Kit, Qiagen, Hilden).

Der mobilisierbare Klonierungsvektor pK18mobsacB wurde mit dem Restriktionsenzym EcoRI verdaut und die Enden mit alkalischer Phosphatase (Alkaline Phosphatase, Boehringer Mannheim, Deutschland) dephosphoryliert. Der so vorbereitete Vektor wurde mit dem ca. 1,6 kb oxyR_A89V-Fragment gemischt und der Ansatz mit T4-DNA-Ligase (Amersham-Pharmacia, Freiburg, Deutschland) behandelt.

Anschließend wurde der E. coli Stamm S17-1 (Simon et al., Bio/Technology 1: 784-791, 1993) mit dem Ligationsansatz transformiert (Hanahan, In. DNA Cloning. A Practical Approach. Vol. 1, ILR-Press, Cold Spring Harbor, New York, 1989). Die Selektion der Plasmid-tragenden Zellen erfolgte durch Ausplattieren des Tansformationsansatztes auf LB-Agar (Sambrook et al., Molecular Cloning: A Laboratory Manual. 2nd Ed., Cold Spring Harbor, New York, 1989), der mit 25 mg/l Kanamycin supplementiert wurde.

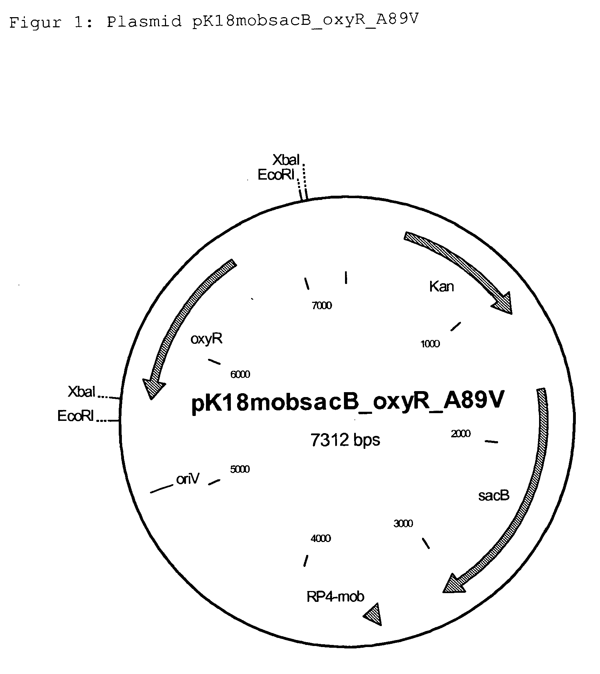

Plasmid-DNA wurde aus einer Transformante mit Hilfe des QIAprep Spin Miniprep Kit der Firma Qiagen isoliert und durch Restriktionsspaltung mit dem Enzym XbaI und anschließender Agarosegel-Elektrophorese überprüft. Das Plasmid wird pK18mobsacB_oxyR_A89V genannt und ist in Figur 1 dargestellt.

### 4.2 Allelaustausch

Der in Beispiel 4.1 genannte Vektor pK18mobsacB_oxyR_A89V wurde nach einem Protokoll von Schäfer et al. (Journal of Microbiology 172: 1663-1666 (1990)) in den C. glutamicum Stamm DM1797 durch Konjugation transferiert. Der Vektor kann in DM1797 nicht selbständig replizieren und bleibt nur dann in der Zelle erhalten, wenn er als Folge eines Rekombinationsereignisses im Chromosom integriert vorliegt. Die Selektion von Transkonjuganten, d. h. von Klonen mit integriertem pK18mobsacB_oxyR_A89V erfolgte durch Ausplattieren des Konjugationsansatzes auf LB-Agar (Sambrook et al., Molecular Cloning: A Laboratory Manual. 2nd Ed. Cold Spring Harbor, New York, 1989), der mit 15 mg/l Kanamycin und 50 mg/l Nalidixinsäure supplementiert wurde. Kanamycin-resistente Transkonjuganten wurden auf LB-Agarplatten mit 25 mg/l Kanamycin ausgestrichen und für 24 Stunden bei 33°C inkubiert. Zur Selektion von Mutanten, bei denen als Folge eines zweiten Rekombinationsereignisses die Exzision des Plasmides stattgefunden hat, wurden die Klone 30 Stunden unselektiv in LB-Flüssigmedium kultiviert, anschließend auf LB-Agar mit 10% Sucrose ausgestrichen und 16 Stunden bebrütet.

Das Plasmid pK18mobsacB_oxyR_A89V enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression führt zur Bildung der Levan-Sucrase, die die Synthese des für C. glutamicum toxischen Produktes Levan katalysiert. Auf LB-Agar mit Sucrose wachsen daher nur solche Klone an, bei denen das integrierte pK18mobsacB_oxyR_A89V als Folge eines zweiten Rekombinationsereignisses exzisiert hat. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Ungefähr 40 bis 50 Kolonien wurden auf den Phänotyp "Wachstum in Gegenwart von Sucrose" und "Nicht-Wachstum in Gegenwart von Kanamycin" geprüft. Bei 4 Kolonien, die den Phänotyp "Wachstum in Gegenwart von Sucrose" und "Nicht-Wachstum in Gegenwart von Kanamycin" aufwiesen, wurde ein die Mutation A89V überspannender Bereich des oxyR-Gens, ausgehend von dem Sequenzierprimer ox1-2 (entspricht der Nukleotidsequenz Position 79-98 der Kodierregion des oxyR-Gens aus SEQ ID NO: 1), von der Firma Agowa (Berlin, Deutschland) sequenziert, um nachzuweisen, dass die Mutation des oxyR_A89V-Allels im Chromosom vorliegt. Der verwendete Primer ox1-2 wurde dazu von der Firma Agowa synthetisiert:
ox1-2:
   5' act gct gcc acc aag ctg tc 3'

Auf diese Weise wurde ein Klon identifiziert, der an der Position 266 der Kodierregion des oxyR-Gens die Base Thymin enthält und somit das oxyR_A89V-Allel besitzt. Dieser Klon wird als Stamm DM1797oxyR_A89V bezeichnet.

### Beispiel 6

Vergleich der Leistung des Stammes DM1797oxyR_A89V mit der des Ausgangsstammes DM1797

Der Leistungstest des in Beispiel 5 erhaltenen C. glutamicum Stammes DM1797oxyR_A89V wurde wie in Beispiel 2 beschrieben durchgeführt. Das Ergebnis des Versuchs ist in Tabelle 2 dargestellt.

**Tabelle 2**

| Stamm | OD (660 nm) | Lysin-HCl g/l |
|---|---|---|
| DM1797 | 9,2 | 2,17 |
| DM1797oxyR_A89V | 9,2 | 2,38 |

### Kurze Beschreibung der Figur:

### Figur 1: Karte des Plasmids pK18mobsacB_oxyR_A89V.

Die verwendeten Abkürzungen und Bezeichnungen haben folgende Bedeutung. Bei der Angabe der Basenpaarzahlen handelt es sich um Näherungswerte, die im Rahmen der Reproduzierbarkeit von Messungen erhalten werden.
- Kan:: Kanamycin Resistenz-Gen
- EcoRI:: Schnittstelle des Restriktionsenzyms EcoRI
- XbaI:: Schnittstelle des Restriktionsenzyms XbaI
- oxyR:: oxyR_A89V-Allel
- sacB:: sacB-Gen
- RP4-mob:: mob-Region mit dem Replikationsursprung für den Transfer (oriT)
- oriV:: Replikationsursprung V

## Patentansprüche

1. Isolierte Mutanten coryneformer Bakterien, welche ein Gen enthalten, das für ein Polypeptid mit OxyR-Transkriptionsregulator-Aktivität kodiert, **dadurch gekennzeichnet, dass** das Polypeptid eine Aminosäuresequenz umfasst, ausgewählt aus der Gruppe:
a) Aminosäuresequenz SEQ ID NO: 6, die durch Austausch von L-Alanin gegen L-Valin an der Position 89 in SEQ ID NO: 2 entstanden ist oder
b) Aminosäuresequenz gemäß SEQ ID NO: 6, die durch Austausch von L-Alanin gegen L-Valin an der Position 89 in SEQ ID NO: 2 entstanden ist und die zusätzlich maximal fünf konservative Aminosäureaustausche und maximal 5 Insertionen oder Deletionen enthält, wobei die maximal fünf konservativen Aminosäureaustausche und maximal 5 Insertionen oder Deletionen die Aktivität im Wesentlichen nicht verändern.

2. Mutanten coryneformer Bakterien gemäß Anspruch 1, **dadurch gekennzeichnet, dass** es sich bei den coryneformen Bakterien um ein Bakterium, ausgewählt aus der Gruppe Corynebacterium efficiens, Corynebacterium glutamicum, Corynebacterium thermoaminogenes und Corynebacterium aminogenes handelt.

3. Mutanten coryneformer Bakterien gemäß Anspruch 2, **dadurch gekennzeichnet, dass** es sich um Corynebacterium glutamicum handelt.

4. Mutanten coryneformer Bakterien gemäß Anspruch 1, 2 oder 3, **dadurch gekennzeichnet, dass** es sich um L-Lysin ausscheidende Bakterien handelt.

5. Mutanten coryneformer Bakterien gemäß Anspruch 1 bis 4 **dadurch gekennzeichnet, dass** das Polypeptid eine Aminosäuresequenz mit einer Länge von 327 L-Aminosäuren umfasst.

6. Mutanten coryneformer Bakterien gemäß Anspruch 1, **dadurch gekennzeichnet, dass** das Gen die Nukleotidsequenz von SEQ ID NO:5 oder SEQ ID NO:7 umfasst, wobei Cytosin an der Position 731 von SEQ ID NO:5 oder Cytosin an der Position 1481 von SEQ ID NO:7 durch Thymin ersetzt ist .

7. Mutanten coryneformer Bakterien gemäß Anspruch 5, **dadurch gekennzeichnet, dass** das Gen die Nukleotidsequenz von SEQ ID NO:7 umfasst.

8. Isoliertes Polynukleotid, das für ein Polypeptid mit OxyR-Transkriptionsregulator Aktivität kodiert, **dadurch gekennzeichnet, dass** das Polypeptid eine Aminosäuresequenz umfasst, ausgewählt aus der Gruppe:
a) Aminosäuresequenz SEQ ID NO: 6, die durch Austausch von L-Alanin gegen L-Valin an der Position 89 in SEQ ID NO: 2 entstanden ist, oder
b) Aminosäuresequenz gemäß SEQ ID NO: 6, die durch Austausch von L-Alanin gegen L-Valin an der Position 89 in SEQ ID NO: 2 entstanden ist und die zusätzlich maximal fünf konservative Aminosäureaustausche und maximal 5 Insertionen oder Deletionen enthält, wobei die maximal fünf konservativen Aminosäureaustausche und maximal 5 Insertionen oder Deletionen die Aktivität im Wesentlichen nicht verändern.

9. Isoliertes Polynukleotid gemäß Anspruch 8, **dadurch gekennzeichnet, dass** es das kodierte Polypeptid einer Aminosäuresequenz mit einer Länge von 327 L-Aminosäuren umfasst.

10. Isoliertes Polynukleotid gemäß Anspruch 8, **dadurch gekennzeichnet, dass** die Nukleotidsequenz SEQ ID NO:5 umfasst, oder SEQ ID NO:5 wobei diese gegebenenfalls zusätzlich einen Basenaustausch an der Position 731 Thymin anstelle von Cytosin umfasst.

11. Isoliertes Polynukleotid gemäß Anspruch 8, **dadurch gekennzeichnet, dass** die Nukleotidsequenz SEQ ID NO:7 umfasst.

12. Ein isoliertes Polynukleotid, welches ein Nukleinsäure-Molekül umfasst, das mindestens für ein Leseraster mit einer Aminosäuresequenz entsprechend Position 70 bis 108 von SEQ ID NO:2 kodiert, wobei an der Position entsprechend 89 von SEQ ID NO:2 L-Valin enthalten ist.

13. Isoliertes Polynukleotid gemäß Anspruch 12, **dadurch gekennzeichnet, dass** es mindestens für ein Leseraster mit einer Aminosäuresequenz entsprechend Position 70 bis 108 von SEQ ID NO:6 kodiert.

14. Isoliertes Polynukleotid gemäß Anspruch 13, **dadurch gekennzeichnet, dass** es mindestens die Nukleotidsequenz entsprechend Position 958 bis 1074 von SEQ ID NO:7 umfasst, wobei gegebenenfalls an Position 731 Thymin enthalten ist.

15. Ein rekombinanter Mikroorganismus, der das isolierte Polynukleotid gemäß Anspruch 8 bis 11 enthält.

16. Rekombinanter Mikroorganismus gemäß Anspruch 15, **dadurch gekennzeichnet, dass** es sich um ein coryneformes Bakterium oder um ein Bakterium der Gattung Escherichia handelt.

17. Rekombinanter Mikroorganismus gemäß Anspruch 16, **dadurch gekennzeichnet, dass** es sich bei dem coryneformen Bakterium um die Gattung Corynebacterium handelt.

18. Rekombinanter Mikroorganismus gemäß Anspruch 17, **dadurch gekennzeichnet, dass** es sich bei dem Bakterium der Gattung Corynebacterium um die Art Corynebacterium glutamicum handelt.

19. Ein Vektor, der das isolierte Polynukleotid gemäß Anspruch 8 bis 11 enthält.

20. Ein rekombinanter Mikroorganismus gemäß Anspruch 18, der den Vektor gemäß Anspruch 19 enthält.

21. Rekombinanter Mikroorganismus, gemäß Anspruch 20, **dadurch gekennzeichnet, dass** es sich um ein coryneformes Bakterium oder um ein Bakterium der Gattung Escherichia handelt.

22. Rekombinanter Mikroorganismus gemäß Anspruch 21, **dadurch gekennzeichnet, dass** es sich bei dem coryneformen Bakterium um die Gattung Corynebacterium handelt.

23. Rekombinanter Mikroorganismus gemäß Anspruch 22, **dadurch gekennzeichnet, dass** es sich bei dem Bakterium der Gattung Corynebacterium um die Art Corynebacterium glutamicum handelt.

24. Verfahren zur Herstellung von L-Lysin **dadurch gekennzeichnet, dass** man
a) ein isoliertes coryneformes Bakterium ausgewählt aus der Gruppe gemäß den Ansprüchen 1-7 und 20-23 in einem geeignetem Medium fermentiert, und
b) L-Lysin in der Fermentationsbrühe oder in den Zellen des Bakteriums anreichert.

25. Verfahren nach Anspruch 24, **dadurch gekennzeichnet, dass** man L-Lysin sammelt oder isoliert.

26. Verfahren nach Anspruch 24, **dadurch gekennzeichnet, dass** man L-Lysin reinigt.

27. Verfahren nach Anspruch 24, **dadurch gekennzeichnet, dass** man L-Lysin gemeinsam mit Bestandteilen aus der Fermentationsbrühe und/oder der Biomasse (> 0 bis 100 % der Biomasse) isoliert oder sammelt.

28. Verfahren nach den Ansprüchen 24 und 27, **dadurch gekennzeichnet, dass** man
a) aus der in Schritt b) von Anspruch 24 erhaltenen Fermentationsbrühe die gebildete Biomasse in einer Menge von 0 bis 100 % entfernt, und
b) aus der in Schritt a) erhaltenen Brühe ein im wesentlichen trockenes und geformtes Produkt durch eine Methode ausgewählt aus der Gruppe Granulation, Kompaktierung, Sprühtrocknung und Extrusion herstellt.

29. Verfahren nach Anspruch 28, **dadurch gekennzeichnet, dass** man L-Lysin herstellt und der Fermentationsbrühe vor oder nach Schritt a) eine Säure ausgewählt aus der Gruppe Schwefelsäure, Salzsäure und Phosphorsäure hinzufügt.

30. Verfahren nach Anspruch 28, **dadurch gekennzeichnet, dass** man aus der vor oder nach in Schritt a) erhaltenen Brühe Wasser entfernt.

31. Verfahren gemäß Schritt 28, **dadurch gekennzeichnet, dass** man das in oder während Schritt b) erhaltene geformte Produkt mit einem Öl besprüht.

## Claims

1. Isolated mutants of coryneform bacteria which comprise a gene which codes for a polypeptide having OxyR transcription regulator activity, **characterized in that** the polypeptide includes an amino acid sequence selected from the group consisting of:
a) amino acid sequence SEQ ID NO: 6 produced by substitution of L-valine for L-alanine at position 89 in SEQ ID NO: 2, or
b) amino acid sequence set forth in SEQ ID NO: 6 produced by substitution of L-valine for L-alanine at position 89 in SEQ ID NO: 2 and additionally comprising no more than five conservative amino acid substitutions and no more than 5 insertions or deletions, said no more than five conservative amino acid substitutions and no more than 5 insertions or deletions not substantially altering the activity.

2. Mutants of coryneform bacteria according to Claim 1, **characterized in that** the coryneform bacteria take the form of a bacterium selected from the group of Corynebacterium efficiens, Corynebacterium glutamicum, Corynebacterium thermoaminogenes and Corynebacterium aminogenes.

3. Mutants of coryneform bacteria according to Claim 2, **characterized in that** they take the form of Corynebacterium glutamicum.

4. Mutants of coryneform bacteria according to Claim 1, 2, or 3, **characterized in that** they are L-lysine-secreting bacteria.

5. Mutants of coryneform bacteria according to Claim 1 to 4, **characterized in that** the polypeptide includes an amino acid sequence having a length of 327 L-amino acids.

6. Mutants of coryneform bacteria according to Claim 1, **characterized in that** the gene includes the nucleotide sequence of SEQ ID NO:5 or SEQ ID NO:7, with cytosine at position 731 of SEQ ID NO:5 or cytosine at position 1481 of SEQ ID NO:7 being replaced by thymine.

7. Mutants of coryneform bacteria according to Claim 5, **characterized in that** the gene includes the nucleotide sequence of SEQ ID NO: 7.

8. Isolated polynucleotide which codes for a polypeptide having OxyR transcription regulator activity, **characterized in that** the polypeptide includes an amino acid sequence selected from the group consisting of:
a) amino acid sequence of SEQ ID NO: 6, produced by substitution of L-valine for L-alanine at position 89 in SEQ ID NO: 2, or
b) amino acid sequence set forth in SEQ ID NO: 6, produced by substitution of L-valine for L-alanine at position 89 in SEQ ID NO: 2 and additionally comprising no more than five conservative amino acid substitutions and no more than 5 insertions or deletions, said no more than five conservative amino acid substitutions and no more than 5 insertions or deletions not substantially altering the activity.

9. Isolated polynucleotide according to Claim 8, **characterized in that** it includes the encoded polypeptide of an amino acid sequence having a length of 327 L-amino acids.

10. Isolated polynucleotide according to Claim 8, **characterized in that** the nucleotide sequence includes SEQ ID NO: 5, or SEQ ID NO: 5 which additionally includes a base substitution of thymine for cytosine at position 731 where appropriate.

11. Isolated polynucleotide according to Claim 8, **characterized in that** the nucleotide sequence includes SEQ ID NO:7.

12. An isolated polynucleotide which includes a nucleic acid molecule which codes at least for a reading frame having an amino acid sequence corresponding to position 70 to 108 of SEQ ID NO:2, where L-valine is present at the position corresponding to 89 of SEQ ID NO:2.

13. Isolated polynucleotide according to Claim 12, **characterized in that** it codes at least for a reading frame having an amino acid sequence corresponding to position 70 to 108 of SEQ ID NO:6.

14. Isolated polynucleotide according to Claim 13, **characterized in that** it includes at least the nucleotide sequence corresponding to position 958 to 1074 of SEQ ID NO:7, with thymine being present at position 731 where appropriate.

15. A recombinant microorganism which comprises the isolated polynucleotide according to Claim 8 to 11.

16. Recombinant microorganism according to Claim 15, **characterized in that** it is a coryneform bacterium or a bacterium of the genus Escherichia.

17. Recombinant microorganism according to Claim 16, **characterized in that** the coryneform bacterium takes the form of the genus Corynebacterium.

18. Recombinant microorganism according to Claim 17, **characterized in that** the bacterium of the genus Corynebacterium takes the form of the species Corynebacterium glutamicum.

19. A vector which comprises the isolated polynucleotide according to Claim 8 to 11.

20. A recombinant microorganism according to Claim 18 which comprises the vector according to Claim 19.

21. Recombinant microorganism according to Claim 20, **characterized in that** it is a coryneform bacterium or a bacterium of the genus Escherichia.

22. Recombinant microorganism according to Claim 21, **characterized in that** the coryneform bacterium takes the form of the genus Corynebacterium.

23. Recombinant microorganism according to Claim 22, **characterized in that** the bacterium of the genus Corynebacterium takes the form of the species Corynebacterium glutamicum.

24. Process for producing L-lysine, **characterized in that**
c) an isolated coryneform bacterium selected from the group according to Claims 1-7 and 20-23 is fermented in a suitable medium, and
d) L-lysine is accumulated in the fermentation broth or in the cells of the bacterium.

25. Process according to Claim 24, **characterized in that** L-lysine is collected or isolated.

26. Process according to Claim 24, **characterized in that** L-lysine is purified.

27. Process according to Claim 24, **characterized in that** L-lysine is isolated or collected together with constituents of the fermentation broth and/or of the biomass (> 0 to 100 % of the biomass).

28. Process according to Claims 24 and 27, **characterized in that**
a) the biomass formed is removed in an amount of from 0 to 100% from the fermentation broth obtained in step b) of Claim 24, and
b) a substantially dry and shaped product is produced, by a method selected from the group of granulation, compaction, spray drying and extrusion, from the broth obtained in step a).

29. Process according to Claim 28, **characterized in that** L-lysine is produced and an acid selected from the group of sulphuric acid, hydrochloric acid and phosphoric acid is added to the fermentation broth before or after step a).

30. Process according to Claim 28, **characterized in that** water is removed from the broth obtained before or after in step a).

31. Process according to step 28, **characterized in that** the shaped product obtained in or during step b) is sprayed with an oil.

## Revendications

1. Mutants isolés de bactéries corynéformes, qui contiennent un gène qui code pour un polypeptide à activité de régulateur de transcription OxyR, **caractérisés en ce que** le polypeptide comprend une séquence d'acides aminés choisie dans le groupe :
a) séquence d'acides aminés SEQ ID NO: 6, qui est obtenue par remplacement de la L-alanine par la L-valine à la position 89 dans SEQ ID NO: 2 ou
b) séquence d'acides aminés selon SEQ ID NO: 6, qui est obtenue par remplacement de la L-alanine par la L-valine à la position 89 dans SEQ ID NO: 2 et qui en plus comporte au maximum cinq remplacements d'acides aminés conservateurs et au maximum 5 insertions ou délétions, les au maximum cinq remplacements d'acides aminés conservateurs et au maximum 5 insertions ou délétions ne modifiant pratiquement pas l'activité.

2. Mutants de bactéries corynéformes selon la revendication 1, **caractérisés en ce que** pour ce qui est des bactéries corynéformes il s'agit d'une bactérie choisie dans le groupe constitué par *Corynebacterium efficiens, Corynebacterium glutamicum, Corynebacterium thermoaminogenes* et *Corynebacterium aminogenes.*

3. Mutants de bactéries corynéformes selon la revendication 2, **caractérisés en ce qu'**il s'agit de *Corynebacterium glutamicum.*

4. Mutants de bactéries corynéformes selon la revendication 1, 2 ou 3, **caractérisés en ce qu'**il s'agit de bactéries excrétant de la L-lysine.

5. Mutants de bactéries corynéformes selon l'une quelconque des revendications 1 à 4, **caractérisés en ce que** le polypeptide comprend une séquence d'acides aminés ayant une longueur de 327 acides aminés L.

6. Mutants de bactéries corynéformes selon la revendication 1, **caractérisés en ce que** le gène comprend la séquence de nucléotides de SEQ ID NO: 5 ou SEQ ID NO: 7, la cytosine à la position 731 de SEQ ID NO: 5 ou la cytosine à la position 1481 de SEQ ID NO: 7 étant remplacée par la thymine.

7. Mutants de bactéries corynéformes selon la revendication 5, **caractérisés en ce que** le gène comprend la séquence de nucléotides de SEQ ID NO: 7.

8. Polynucléotide isolé, qui code pour un polypeptide à activité de régulateur de transcription OxyR, **caractérisé en ce que** le polypeptide comprend une séquence d'acides aminés choisie dans le groupe :
a) séquence d'acides aminés SEQ ID NO: 6, qui est obtenue par remplacement de la L-alanine par la L-valine à la position 89 dans SEQ ID NO: 2, ou
b) séquence d'acides aminés selon SEQ ID NO: 6, qui est obtenue par remplacement de la L-alanine par la L-valine à la position 89 dans SEQ ID NO: 2 et qui en plus comporte au maximum cinq remplacements d'acides aminés conservateurs et au maximum 5 insertions ou délétions, les au maximum cinq remplacements d'acides aminés conservateurs et au maximum 5 insertions ou délétions ne modifiant pratiquement pas l'activité.

9. Polynucléotide isolé selon la revendication 8, **caractérisé en ce qu'**il comprend le polypeptide codé d'une séquence d'acides aminés ayant une longueur de 327 acides aminés L.

10. Polynucléotide isolé selon la revendication 8, **caractérisé en ce que** la séquence de nucléotides comprend SEQ ID NO: 5, ou SEQ ID NO: 5 qui éventuellement comprend en outre un remplacement de base à la position 731, thymine au lieu de cytosine.

11. Polynucléotide isolé selon la revendication 8, **caractérisé en ce que** la séquence de nucléotides comprend SEQ ID NO: 7.

12. Polynucléotide isolé, qui comprend une molécule d'acide nucléique qui code au moins pour un cadre de lecture ayant une séquence d'acides aminés correspondant aux positions 70 à 108 de SEQ ID NO: 2, un résidu L-valine étant contenu à la position correspondant à 89 de SEQ ID NO: 2.

13. Polynucléotide isolé selon la revendication 12, **caractérisé en ce qu'** il code au moins pour un cadre de lecture ayant une séquence d'acides aminés correspondant aux positions 70 à 108 de SEQ ID NO: 6.

14. Polynucléotide isolé selon la revendication 13, **caractérisé en ce qu'**il comprend au moins la séquence de nucléotides correspondant aux positions 958 à 1074 de SEQ ID NO: 7, éventuellement une thymine étant contenue à la position 731.

15. Micro-organisme recombinant, qui contient le polynucléotide isolé selon l'une quelconque des revendications 8 à 11.

16. Micro-organisme recombinant selon la revendication 15, **caractérisé en ce qu'**il s'agit d'une bactérie corynéforme ou d'une bactérie appartenant au genre *Escherichia.*

17. Micro-organisme recombinant selon la revendication 16, **caractérisé en ce que** pour ce qui est de la bactérie corynéforme il s'agit du genre *Corynebacterium.*

18. Micro-organisme recombinant selon la revendication 17, **caractérisé en ce que** pour ce qui est de la bactérie appartenant au gendre *Corynebacterium* il s'agit de l'espèce *Corynebacterium glutamicum.*

19. Vecteur, qui contient le polynucléotide isolé selon l'une quelconque des revendications 8 à 11.

20. Micro-organisme recombinant selon la revendication 18, qui contient le vecteur selon la revendication 19.

21. Micro-organisme recombinant selon la revendication 20, **caractérisé en ce qu'**il s'agit d'une bactérie corynéforme ou d'une bactérie appartenant au genre *Escherichia.*

22. Micro-organisme recombinant selon la revendication 21, **caractérisé en ce que** pour ce qui est de la bactérie corynéforme il s'agit du genre *Corynebacterium.*

23. Micro-organisme recombinant selon la revendication 22, **caractérisé en ce que** pour ce qui est de la bactérie appartenant au genre *Corynebacterium* il s'agit de l'espèce *Corynebacterium glutamicum.*

24. Procédé pour la production de L-lysine, **caractérisé en ce que**
a) on cultivé par fermentation dans un milieu approprié une bactérie corynéforme isolée, choisie dans le groupe selon les revendications 1-7 et 20-23, et
b) de la L-lysine s'accumule dans le bouillon de fermentation ou dans les cellules de la bactérie.

25. Procédé selon la revendication 24, **caractérisé en ce qu'**on recueille ou isole la L-lysine.

26. Procédé selon la revendication 24, **caractérisé en ce qu'**on purifie la L-lysine.

27. Procédé selon la revendication 24, **caractérisé en ce que** qu'on isole ou recueille de la L-lysine conjointement avec des composants provenant du bouillon se fermentation et/ou la biomasse (> 0 à 100 % de la biomasse).

28. Procédé selon les revendications 24 et 27, **caractérisé en ce que**
a) on sépare du bouillon de fermentation obtenu dans l'étape b) de la revendication 24 la biomasse formée, en une quantité de 0 à 100 %, et
b) on prépare à partir du bouillon obtenu dans l'étape a) un produit moulé et pratiquement sec, par une méthode choisie dans le groupe constitué par la granulation, le compactage, le séchage par atomisation et l'extrusion.

29. Procédé selon la revendication 28, **caractérisé en ce qu'**on produit de la L-lysine et on ajoute au bouillon de fermentation, avant ou après l'étape a), un acide choisi dans le groupe constitué par l'acide sulfurique, l'acide chlorhydrique et l'acide phosphorique.

30. Procédé selon la revendication 28, **caractérisé en ce qu'**on élimine l'eau du bouillon obtenu avant ou après dans l'étape a).

31. Procédé selon l'étape 28, **caractérisé en ce qu'**on pulvérise une huile sur le produit moulé obtenu dans ou pendant l'étape b).


### SEQUENCE LISTING

<110> Evonik Degussa GmbH
<120> Allele des oxyR-Gens coryneformer Bakterien
<130> 200600763
<160> 24
<170> PatentIn version 3.3
<210> 1
   <211> 984
   <212> DNA
   <213> Corynebacterium glutamicum
<220>
   <221> CDS
   <222> (1)..(981)
   <223> oxyR-Wildtyp-Gen
<220>
   <221> misc_feature
   <222> (266)..(266)
   <223> Cytosin
<220>
   <221> misc_feature
   <222> (731)..(731)
   <223> Cytosin
<400> 1
   atg agc aat aaa gag tac cgg ccc aca ctc gcc cag ctt cgc acc ttt      48
   Met Ser Asn Lys Glu Tyr Arg Pro Thr Leu Ala Gln Leu Arg Thr Phe
   1               5                   10                  15
   gtc acc atc gca gaa tgc aag cac ttt ggt act gct gcc acc aag ctg      96
   Val Thr Ile Ala Glu Cys Lys His Phe Gly Thr Ala Ala Thr Lys Leu
               20                  25                  30
   tcc att tcg cag cca tcc ctc tcc cag gca ctt gtc gca tta gaa aca     144
   Ser Ile Ser Gln Pro Ser Leu Ser Gln Ala Leu Val Ala Leu Glu Thr
           35                  40                  45
   ggc ctg gga gtt cag ctg att gaa cgc tcc acc cgc aag gtc att gtc     192
   Gly Leu Gly Val Gln Leu Ile Glu Arg Ser Thr Arg Lys Val Ile Val
       50                  55                  60
   acc cca gcg ggc gag aag ttg ctg cca ttc gcc aaa tcc acc ctt gac     240
   Thr Pro Ala Gly Glu Lys Leu Leu Pro Phe Ala Lys Ser Thr Leu Asp
   65                  70                  75                  80
   gcg gcg gag tct ttc ctc tcc cac gcc aag ggc gcc aac ggt tcg ctc     288
   Ala Ala Glu Ser Phe Leu Ser His Ala Lys Gly Ala Asn Gly Ser Leu
                   85                  90                  95
   act gga ccg ttg acc gta ggc atc atc ccc acg gcg gct cct tac att     336
   Thr Gly Pro Leu Thr Val Gly Ile Ile Pro Thr Ala Ala Pro Tyr Ile
               100                 105                 110
   ttg ccg tca atg ctg tcc atc gtg gat gaa gaa tat cca gat ctg gaa     384
   Leu Pro Ser Met Leu Ser Ile Val Asp Glu Glu Tyr Pro Asp Leu Glu
           115                 120                 125
   cct cac atc gtc gag gac caa acc aag cat ctt ctc gcg ttg ctg cgc     432
   Pro His Ile Val Glu Asp Gln Thr Lys His Leu Leu Ala Leu Leu Arg
       130                 135                 140
   gac ggc gcc atc gac gtc gcc atg atg gcc ctg cct tct gag gca cca      480
   Asp Gly Ala Ile Asp Val Ala Met Met Ala Leu Pro Ser Glu Ala Pro
   145                 150                 155                 160
   ggc atg aag gaa atc ccc ctc tac gac gaa gac ttt atc gtc gtt aca      528
   Gly Met Lys Glu Ile Pro Leu Tyr Asp Glu Asp Phe Ile Val Val Thr
                   165                 170                 175
   gct agc gat cac ccc ttc gcc ggc cgc caa gac tta gaa cta tcc gcc      576
   Ala Ser Asp His Pro Phe Ala Gly Arg Gln Asp Leu Glu Leu Ser Ala
               180                 185                 190
   tta gaa gac ctc gat ctg ctg ctt ctc gac gac gga cac tgc ctc cac      624
   Leu Glu Asp Leu Asp Leu Leu Leu Leu Asp Asp Gly His Cys Leu His
           195                 200                 205
   gac caa att gtg gac ctg tgc cgc cgc gga gac atc aac ccc att agc      672
   Asp Gln Ile Val Asp Leu Cys Arg Arg Gly Asp Ile Asn Pro Ile Ser
       210                 215                 220
   tcc act act gct gtc acc cgc gca tcc agc ctt acc acc gtc atg cag      720
   Ser Thr Thr Ala Val Thr Arg Ala Ser Ser Leu Thr Thr Val Met Gln
   225                 230                 235                 240
   ctc gtc gtc gcc ggc ctt gga tcc acc ttg gtc cca atc agc gca atc      768
   Leu Val Val Ala Gly Leu Gly Ser Thr Leu Val Pro Ile Ser Ala Ile
                   245                 250                 255
   cca tgg gaa tgc acc cga cca gga ctg gca aca gcc aac ttc aac tct      816
   Pro Trp Glu Cys Thr Arg Pro Gly Leu Ala Thr Ala Asn Phe Asn Ser
               260                 265                 270
   gat gtc acc gca aac cgc cgc att gga ttg gtg tac cgt tcc tct tct      864
   Asp Val Thr Ala Asn Arg Arg Ile Gly Leu Val Tyr Arg Ser Ser Ser
           275                 280                 285
   tct cgc gcc gaa gag ttc gaa cag ttt gca ctc att ttg cag cgc gct      912
   Ser Arg Ala Glu Glu Phe Glu Gln Phe Ala Leu Ile Leu Gln Arg Ala
       290                 295                 300
   ttc caa gaa gcc gtc gcg ctt gct gcc tca act ggc atc acc ttg aag      960
   Phe Gln Glu Ala Val Ala Leu Ala Ala Ser Thr Gly Ile Thr Leu Lys
   305                 310                 315                 320
   caa aat gtc gcg gta gcg cag taa                                      984
   Gln Asn Val Ala Val Ala Gln
                   325
<210> 2
   <211> 327
   <212> PRT
   <213> Corynebacterium glutamicum
<400> 2
   Met Ser Asn Lys Glu Tyr Arg Pro Thr Leu Ala Gln Leu Arg Thr Phe
   1               5                   10                  15
   Val Thr Ile Ala Glu Cys Lys His Phe Gly Thr Ala Ala Thr Lys Leu
               20                  25                  30
   Ser Ile Ser Gln Pro Ser Leu Ser Gln Ala Leu Val Ala Leu Glu Thr
           35                  40                  45
   Gly Leu Gly Val Gln Leu Ile Glu Arg Ser Thr Arg Lys Val Ile Val
   50 55 60
   Thr Pro Ala Gly Glu Lys Leu Leu Pro Phe Ala Lys Ser Thr Leu Asp
   65 70 75 80
   Ala Ala Glu Ser Phe Leu Ser His Ala Lys Gly Ala Asn Gly Ser Leu
   85 90 95
   Thr Gly Pro Leu Thr Val Gly Ile Ile Pro Thr Ala Ala Pro Tyr Ile
   100 105 110
   Leu Pro Ser Met Leu Ser Ile Val Asp Glu Glu Tyr Pro Asp Leu Glu
   115 120 125
   Pro His Ile Val Glu Asp Gln Thr Lys His Leu Leu Ala Leu Leu Arg
   130 135 140
   Asp Gly Ala Ile Asp Val Ala Met Met Ala Leu Pro Ser Glu Ala Pro
   145 150 155 160
   Gly Met Lys Glu Ile Pro Leu Tyr Asp Glu Asp Phe Ile Val Val Thr
   165 170 175
   Ala Ser Asp His Pro Phe Ala Gly Arg Gln Asp Leu Glu Leu Ser Ala
   180 185 190
   Leu Glu Asp Leu Asp Leu Leu Leu Leu Asp Asp Gly His Cys Leu His
   195 200 205
   Asp Gln Ile Val Asp Leu Cys Arg Arg Gly Asp Ile Asn Pro Ile Ser
   210 215 220
   Ser Thr Thr Ala Val Thr Arg Ala Ser Ser Leu Thr Thr Val Met Gln
   225 230 235 240
   Leu Val Val Ala Gly Leu Gly Ser Thr Leu Val Pro Ile Ser Ala Ile
   245 250 255
   Pro Trp Glu Cys Thr Arg Pro Gly Leu Ala Thr Ala Asn Phe Asn Ser
   260 265 270
   Asp Val Thr Ala Asn Arg Arg Ile Gly Leu Val Tyr Arg Ser Ser Ser
   275 280 285
   Ser Arg Ala Glu Glu Phe Glu Gln Phe Ala Leu Ile Leu Gln Arg Ala
   290 295 300
   Phe Gln Glu Ala Val Ala Leu Ala Ala Ser Thr Gly Ile Thr Leu Lys
   305 310 315 320
   Gln Asn Val Ala Val Ala Gln
   325
<210> 3
   <211> 2484
   <212> DNA
   <213> Corynebacterium glutamicum
<220>
   <221> misc_feature
   <222> (1)..(750)
   <223> stromaufwärts der CDS gelegene Nukleotidsequenz
<220>
   <221> CDS
   <222> (751)..(1731)
   <223> oxyR-wildtyp-Gen
<220>
   <221> misc_feature
   <222> (1016)..(1016)
   <223> Cytosin
<220>
   <221> misc_feature
   <222> (1481)..(1481)
   <223> Cytosin
<220>
   <221> misc_feature
   <222> (1732)..(2484)
   <223> stromabwärts der CDS gelegene Nukleotidsequenz
<400> 3
   cctacggtgt ggccaggtca gagggtgttg cgctgcgcta cctcaccgat gcctggcgta      60
   ctctccaaca ctcactgcct acggaagctc gcaccgagga acttgatgat gttgtggtgt     120
   ggttgggcga actgatcaaa caggtcgact cctccctggt ggatgagtgg gctcagatgg     180
   cggatccaga agcaccgatc agcaaggaag cgctggaacg agagctcgcc ttcggcgtgg     240
   aagacccaac agcacttacc gccaaccgca gggcatttac catcatggtg cgcaacgcca     300
   tgttccgcct tgtggagcta tttgcttatg aaaaggaaga tcagcttagt cagatgactg     360
   aatacctgga tgaggctcct gatttcggtg ctgcgatgga tgcgtacttt gatgaatatg     420
   cggatcttga taccggcccg gcagctcgtg gaccagagtt cttcaaggta gagcacacgg     480
   gaagaatgtg ggaggtgcgt caggtggtga aggatccaga aggtgataat tccttcgcgt     540
   ttgttgccac cattgatctt gatgcctctg atgatgcagg tgaggtgcgt tttggatcgc     600
   tgtcgattga ccacaactag gggtttgcgt cgaaaagcaa gcacgcctgg tgcctgattt      660
   gagcggtttt acctatggcg ctttggcgcc gtcaaactgt cccagcgatt tcattattat     720
   tttcgtgcat tcaccgttat agttataggc atg agc aat aaa gag tac cgg ccc      774
                                     Met Ser Asn Lys Glu Tyr Arg Pro
                                     1               5
   aca ctc gcc cag ctt cgc acc ttt gtc acc atc gca gaa tgc aag cac      822
   Thr Leu Ala Gln Leu Arg Thr Phe Val Thr Ile Ala Glu Cys Lys His
       10                  15                  20
   ttt ggt act gct gcc acc aag ctg tcc att tcg cag cca tcc ctc tcc      870
   Phe Gly Thr Ala Ala Thr Lys Leu Ser Ile Ser Gln Pro Ser Leu Ser
   25                  30                  35                  40
   cag gca ctt gtc gca tta gaa aca ggc ctg gga gtt cag ctg att gaa     918
   Gln Ala Leu Val Ala Leu Glu Thr Gly Leu Gly Val Gln Leu Ile Glu
                   45                  50                  55
   cgc tcc acc cgc aag gtc att gtc acc cca gcg ggc gag aag ttg ctg     966
   Arg Ser Thr Arg Lys Val Ile Val Thr Pro Ala Gly Glu Lys Leu Leu
               60                  65                  70
   cca ttc gcc aaa tcc acc ctt gac gcg gcg gag tct ttc ctc tcc cac    1014
   Pro Phe Ala Lys Ser Thr Leu Asp Ala Ala Glu Ser Phe Leu Ser His
           75                  80                  85
   gcc aag ggc gcc aac ggt tcg ctc act gga ccg ttg acc gta ggc atc    1062
   Ala Lys Gly Ala Asn Gly Ser Leu Thr Gly Pro Leu Thr Val Gly Ile
       90                  95                  100
   atc ccc acg gcg gct cct tac att ttg ccg tca atg ctg tcc atc gtg    1110
   Ile Pro Thr Ala Ala Pro Tyr Ile Leu Pro Ser Met Leu Ser Ile Val
   105                 110                 115                 120
   gat gaa gaa tat cca gat ctg gaa cct cac atc gtc gag gac caa acc    1158
   Asp Glu Glu Tyr Pro Asp Leu Glu Pro His Ile Val Glu Asp Gln Thr
                   125                 130                 135
   aag cat ctt ctc gcg ttg ctg cgc gac ggc gcc atc gac gtc gcc atg    1206
   Lys His Leu Leu Ala Leu Leu Arg Asp Gly Ala Ile Asp Val Ala Met
               140                 145                 150
   atg gcc ctg cct tct gag gca cca ggc atg aag gaa atc ccc ctc tac    1254
   Met Ala Leu Pro Ser Glu Ala Pro Gly Met Lys Glu Ile Pro Leu Tyr
           155                 160                 165
   gac gaa gac ttt atc gtc gtt aca gct agc gat cac ccc ttc gcc ggc    1302
   Asp Glu Asp Phe Ile Val Val Thr Ala Ser Asp His Pro Phe Ala Gly
       170                 175                 180
   cgc caa gac tta gaa cta tcc gcc tta gaa gac ctc gat ctg ctg ctt    1350
   Arg Gln Asp Leu Glu Leu Ser Ala Leu Glu Asp Leu Asp Leu Leu Leu
   185                 190                 195                 200
   ctc gac gac gga cac tgc ctc cac gac caa att gtg gac ctg tgc cgc    1398
   Leu Asp Asp Gly His Cys Leu His Asp Gln Ile Val Asp Leu Cys Arg
                   205                 210                 215
   cgc gga gac atc aac ccc att agc tcc act act gct gtc acc cgc gca    1446
   Arg Gly Asp Ile Asn Pro Ile Ser Ser Thr Thr Ala Val Thr Arg Ala
               220                 225                 230
   tcc agc ctt acc acc gtc atg cag ctc gtc gtc gcc ggc ctt gga tcc    1494
   Ser Ser Leu Thr Thr Val Met Gln Leu Val Val Ala Gly Leu Gly Ser
           235                 240                 245
   acc ttg gtc cca atc agc gca atc cca tgg gaa tgc acc cga cca gga    1542
   Thr Leu Val Pro Ile Ser Ala Ile Pro Trp Glu Cys Thr Arg Pro Gly
       250                 255                 260
   ctg gca aca gcc aac ttc aac tct gat gtc acc gca aac cgc cgc att    1590
   Leu Ala Thr Ala Asn Phe Asn Ser Asp Val Thr Ala Asn Arg Arg Ile
   265                 270                 275                 280
   gga ttg gtg tac cgt tcc tct tct tct cgc gcc gaa gag ttc gaa cag    1638
   Gly Leu Val Tyr Arg Ser Ser Ser Ser Arg Ala Glu Glu Phe Glu Gln
                   285                 290                 295
   ttt gca ctc att ttg cag cgc gct ttc caa gaa gcc gtc gcg ctt gct    1686
   Phe Ala Leu Ile Leu Gln Arg Ala Phe Gln Glu Ala Val Ala Leu Ala
               300                 305                 310
   gcc tca act ggc atc acc ttg aag caa aat gtc gcg gta gcg cag        1731
   Ala Ser Thr Gly Ile Thr Leu Lys Gln Asn Val Ala Val Ala Gln
           315                 320                 325
   taagtttttc tagaggtttt ccagagtcag ctacaagcaa aaagcccttt ccattgatgc   1791
   acaccaacgt gagattcaag ggaaagggct ttattgattg cagaatgcct actgcattag   1851
   cggcgctcca ccggaatatt tccaccactg atctggcggt aaatatgaac ggtagacagc   1911
   atcattactg gcagcacgat gatcaggccg aaaccgatcg tgaaaatacc gatgatgagt   1971
   gtggcaaacc cgccaaccag ggagaacagc aacaacattg ggtagttgcg cagcgagtcc   2031
   ttgaaaccag tttgaacagc gcttcctgca gtgtggtgac catcagctgt gtagtacacc   2091
   cagtaggagt acagaggatt gatgaagaac aacaccaggt agacgatgaa gaacatgcct   2151
   aaaccgctgt tattgacctc aacggtaccg gcagcgtcat taaacgacac cagattttga   2211
   gtgagaaaag tggtgaaggt gcccaggatg atgccgaaga tacccagccc caccatcaga   2271
   atcactgttt gaccaacatt gatgggttta aagaaatcac cgaagcgaac tttgtgtcca   2331
   tcaacagaaa gcagtgcacc gcgcatgacg caaatggtta ttgcgaaggt gatgattccg   2391
   atagctacgt tcaacagggt ctcggaaacc gaaaaaccag aagtcgtcgt gcccgcatta   2451
   gggtcgatca aaaatgataa gtagccaagc aac                                2484
<210> 4
   <211> 327
   <212> PRT
   <213> Corynebacterium glutamicum
<400> 4
   Met Ser Asn Lys Glu Tyr Arg Pro Thr Leu Ala Gln Leu Arg Thr Phe
   1 5 10 15
   Val Thr Ile Ala Glu Cys Lys His Phe Gly Thr Ala Ala Thr Lys Leu
   20 25 30
   Ser Ile Ser Gln Pro Ser Leu Ser Gln Ala Leu Val Ala Leu Glu Thr
   35 40 45
   Gly Leu Gly Val Gln Leu Ile Glu Arg Ser Thr Arg Lys Val Ile Val
   50 55 60
   Thr Pro Ala Gly Glu Lys Leu Leu Pro Phe Ala Lys Ser Thr Leu Asp
   65 70 75 80
   Ala Ala Glu Ser Phe Leu Ser His Ala Lys Gly Ala Asn Gly Ser Leu
   85 90 95
   Thr Gly Pro Leu Thr Val Gly Ile Ile Pro Thr Ala Ala Pro Tyr Ile
   100 105 110
   Leu Pro Ser Met Leu Ser Ile Val Asp Glu Glu Tyr Pro Asp Leu Glu
   115 120 125
   Pro His Ile Val Glu Asp Gln Thr Lys His Leu Leu Ala Leu Leu Arg
   130 135 140
   Asp Gly Ala Ile Asp Val Ala Met Met Ala Leu Pro Ser Glu Ala Pro
   145 150 155 160
   Gly Met Lys Glu Ile Pro Leu Tyr Asp Glu Asp Phe Ile Val Val Thr
   165 170 175
   Ala Ser Asp His Pro Phe Ala Gly Arg Gln Asp Leu Glu Leu Ser Ala
   180 185 190
   Leu Glu Asp Leu Asp Leu Leu Leu Leu Asp Asp Gly His Cys Leu His
   195 200 205
   Asp Gln Ile Val Asp Leu Cys Arg Arg Gly Asp Ile Asn Pro Ile Ser
   210 215 220
   Ser Thr Thr Ala Val Thr Arg Ala Ser Ser Leu Thr Thr Val Met Gln
   225 230 235 240
   Leu Val Val Ala Gly Leu Gly Ser Thr Leu Val Pro Ile Ser Ala Ile
   245 250 255
   Pro Trp Glu Cys Thr Arg Pro Gly Leu Ala Thr Ala Asn Phe Asn Ser
   260 265 270
   Asp Val Thr Ala Asn Arg Arg Ile Gly Leu Val Tyr Arg Ser Ser Ser
   275 280 285
   Ser Arg Ala Glu Glu Phe Glu Gln Phe Ala Leu Ile Leu Gln Arg Ala
   290 295 300
   Phe Gln Glu Ala Val Ala Leu Ala Ala Ser Thr Gly Ile Thr Leu Lys
   305 310 315 320
   Gln Asn Val Ala Val Ala Gln
   325
<210> 5
   <211> 984
   <212> DNA
   <213> corynebacterium glutamicum
<220>
   <221> CDS
   <222> (1)..(981)
   <223> oxyR-Allel
<220>
   <221> mutation
   <222> (266)..(266)
   <223> C -> T Transition
<220>
   <221> misc_feature
   <222> (731)..(731)
   <223> Cytosin
<400> 5
   atg agc aat aaa gag tac cgg ccc aca ctc gcc cag ctt cgc acc ttt      48
   Met Ser Asn Lys Glu Tyr Arg Pro Thr Leu Ala Gln Leu Arg Thr Phe
   1               5                   10                  15
   gtc acc atc gca gaa tgc aag cac ttt ggt act gct gcc acc aag ctg      96
   Val Thr Ile Ala Glu Cys Lys His Phe Gly Thr Ala Ala Thr Lys Leu
               20                  25                  30
   tcc att tcg cag cca tcc ctc tcc cag gca ctt gtc gca tta gaa aca      144
   Ser Ile Ser Gln Pro Ser Leu Ser Gln Ala Leu Val Ala Leu Glu Thr
           35                  40                  45
   ggc ctg gga gtt cag ctg att gaa cgc tcc acc cgc aag gtc att gtc      192
   Gly Leu Gly Val Gln Leu Ile Glu Arg Ser Thr Arg Lys Val Ile Val
       50                  55                  60
   acc cca gcg ggc gag aag ttg ctg cca ttc gcc aaa tcc acc ctt gac      240
   Thr Pro Ala Gly Glu Lys Leu Leu Pro Phe Ala Lys Ser Thr Leu Asp
   65                  70                  75                  80
   gcg gcg gag tct ttc ctc tcc cac gtc aag ggc gcc aac ggt tcg ctc      288
   Ala Ala Glu Ser Phe Leu Ser His Val Lys Gly Ala Asn Gly Ser Leu
                   85                  90                  95
   act gga ccg ttg acc gta ggc atc atc ccc acg gcg gct cct tac att      336
   Thr Gly Pro Leu Thr Val Gly Ile Ile Pro Thr Ala Ala Pro Tyr Ile
               100                 105                 110
   ttg ccg tca atg ctg tcc atc gtg gat gaa gaa tat cca gat ctg gaa      384
   Leu Pro Ser Met Leu Ser Ile Val Asp Glu Glu Tyr Pro Asp Leu Glu
           115                 120                 125
   cct cac atc gtc gag gac caa acc aag cat ctt ctc gcg ttg ctg cgc      432
   Pro His Ile Val Glu Asp Gln Thr Lys His Leu Leu Ala Leu Leu Arg
       130                 135                 140
   gac ggc gcc atc gac gtc gcc atg atg gcc ctg cct tct gag gca cca      480
   Asp Gly Ala Ile Asp Val Ala Met Met Ala Leu Pro Ser Glu Ala Pro
   145                 150                 155                 160
   ggc atg aag gaa atc ccc ctc tac gac gaa gac ttt atc gtc gtt aca      528
   Gly Met Lys Glu Ile Pro Leu Tyr Asp Glu Asp Phe Ile Val Val Thr
                   165                 170                 175
   gct agc gat cac ccc ttc gcc ggc cgc caa gac tta gaa cta tcc gcc      576
   Ala Ser Asp His Pro Phe Ala Gly Arg Gln Asp Leu Glu Leu Ser Ala
               180                 185                 190
   tta gaa gac ctc gat ctg ctg ctt ctc gac gac gga cac tgc ctc cac      624
   Leu Glu Asp Leu Asp Leu Leu Leu Leu Asp Asp Gly His Cys Leu His
           195                 200                 205
   gac caa att gtg gac ctg tgc cgc cgc gga gac atc aac ccc att agc      672
   Asp Gln Ile Val Asp Leu Cys Arg Arg Gly Asp Ile Asn Pro Ile Ser
       210                 215                 220
   tcc act act gct gtc acc cgc gca tcc agc ctt acc acc gtc atg cag      720
   Ser Thr Thr Ala Val Thr Arg Ala Ser Ser Leu Thr Thr Val Met Gln
   225                 230                 235                 240
   ctc gtc gtc gcc ggc ctt gga tcc acc ttg gtc cca atc agc gca atc      768
   Leu Val Val Ala Gly Leu Gly Ser Thr Leu Val Pro Ile Ser Ala Ile
                             245                     250                     255
   cca tgg gaa tgc acc cga cca gga ctg gca aca gcc aac ttc aac tct      816
   Pro Trp Glu Cys Thr Arg Pro Gly Leu Ala Thr Ala Asn Phe Asn Ser
               260                     265                     270
   gat gtc acc gca aac cgc cgc att gga ttg gtg tac cgt tcc tct tct      864
   Asp Val Thr Ala Asn Arg Arg Ile Gly Leu Val Tyr Arg Ser Ser Ser
           275                     280                     285
   tct cgc gcc gaa gag ttc gaa cag ttt gca ctc att ttg cag cgc gct      912
   Ser Arg Ala Glu Glu Phe Glu Gln Phe Ala Leu Ile Leu Gln Arg Ala
       290                     295                     300
   ttc caa gaa gcc gtc gcg ctt gct gcc tca act ggc atc acc ttg aag      960
   Phe Gln Glu Ala Val Ala Leu Ala Ala Ser Thr Gly Ile Thr Leu Lys
   305                     310                     315                     320
   caa aat gtc gcg gta gcg cag taa                                      984
   Gln Asn Val Ala Val Ala Gln
                   325
<210> 6
   <211> 327
   <212> PRT
   <213> Corynebacterium glutamicum
<400> 6
   Met Ser Asn Lys Glu Tyr Arg Pro Thr Leu Ala Gln Leu Arg Thr Phe
   1               5                   10                  15
   Val Thr Ile Ala Glu Cys Lys His Phe Gly Thr Ala Ala Thr Lys Leu
               20                  25                  30
   Ser Ile Ser Gln Pro Ser Leu Ser Gln Ala Leu Val Ala Leu Glu Thr
           35                  40                  45
   Gly Leu Gly Val Gln Leu Ile Glu Arg Ser Thr Arg Lys Val Ile Val
       50                  55                  60
   Thr Pro Ala Gly Glu Lys Leu Leu Pro Phe Ala Lys Ser Thr Leu Asp
   65                  70                  75                  80
   Ala Ala Glu Ser Phe Leu Ser His Val Lys Gly Ala Asn Gly Ser Leu
                   85                  90                  95
   Thr Gly Pro Leu Thr Val Gly Ile Ile Pro Thr Ala Ala Pro Tyr Ile
               100                 105                 110
   Leu Pro Ser Met Leu Ser Ile Val Asp Glu Glu Tyr Pro Asp Leu Glu
           115                 120                 125
   Pro His Ile Val Glu Asp Gln Thr Lys His Leu Leu Ala Leu Leu Arg
       130                 135                 140
   Asp Gly Ala Ile Asp Val Ala Met Met Ala Leu Pro Ser Glu Ala Pro
   145                 150                 155                 160
   Gly Met Lys Glu Ile Pro Leu Tyr Asp Glu Asp Phe Ile Val Val Thr
                   165                 170                 175
   Ala Ser Asp His Pro Phe Ala Gly Arg Gln Asp Leu Glu Leu Ser Ala
               180                 185                 190
   Leu Glu Asp Leu Asp Leu Leu Leu Leu Asp Asp Gly His Cys Leu His
           195                 200                 205
   Asp Gln Ile Val Asp Leu Cys Arg Arg Gly Asp Ile Asn Pro Ile Ser
       210                 215                 220
   Ser Thr Thr Ala Val Thr Arg Ala Ser Ser Leu Thr Thr Val Met Gln
   225                 230                 235                 240
   Leu Val Val Ala Gly Leu Gly Ser Thr Leu Val Pro Ile Ser Ala Ile
                   245                 250                 255
   Pro Trp Glu Cys Thr Arg Pro Gly Leu Ala Thr Ala Asn Phe Asn Ser
               260                 265                 270
   Asp Val Thr Ala Asn Arg Arg Ile Gly Leu Val Tyr Arg Ser Ser Ser
           275                 280                 285
   Ser Arg Ala Glu Glu Phe Glu Gln Phe Ala Leu Ile Leu Gln Arg Ala
       290                 295                 300
   Phe Gln Glu Ala Val Ala Leu Ala Ala Ser Thr Gly Ile Thr Leu Lys
   305                 310                 315                 320
   Gln Asn Val Ala Val Ala Gln
                   325
<210> 7
   <211> 2484
   <212> DNA
   <213> Corynebacterium glutamicum
<220>
   <221> misc_feature
   <222> (1)..(750)
   <223> stromaufwärts der CDS gelegene Nukleotidsequenz
<220>
   <221> primer_bind
   <222> (271)..(290)
   <223> Bindungsstelle Primer oxyR_XL_A1
<220>
   <221> CDS
   <222> (751)..(1731)
   <223> oxyR-Allel
<220>
   <221> mutation
   <222> (1016)..(1016)
   <223> C -> T Transition
<220>
   <221> misc_feature
   <222> (1481)..(1481)
   <223> Cytosin
<220>
   <221> misc_feature
   <222> (1732)..(2484)
   <223> stromabwärts der CDS gelegene Nukleotidsequenz
<220>
   <221> primer_bind
   <222> (1834)..(1853)
   <223> Bindungsstelle Primer oxyR_XL_E1
<400> 7
   cctacggtgt ggccaggtca gagggtgttg cgctgcgcta cctcaccgat gcctggcgta     60
   ctctccaaca ctcactgcct acggaagctc gcaccgagga acttgatgat gttgtggtgt    120
   ggttgggcga actgatcaaa caggtcgact cctccctggt ggatgagtgg gctcagatgg    180
   cggatccaga agcaccgatc agcaaggaag cgctggaacg agagctcgcc ttcggcgtgg    240
   aagacccaac agcacttacc gccaaccgca gggcatttac catcatggtg cgcaacgcca    300
   tgttccgcct tgtggagcta tttgcttatg aaaaggaaga tcagcttagt cagatgactg    360
   aatacctgga tgaggctcct gatttcggtg ctgcgatgga tgcgtacttt gatgaatatg    420
   cggatcttga taccggcccg gcagctcgtg gaccagagtt cttcaaggta gagcacacgg    480
   gaagaatgtg ggaggtgcgt caggtggtga aggatccaga aggtgataat tccttcgcgt    540
   ttgttgccac cattgatctt gatgcctctg atgatgcagg tgaggtgcgt tttggatcgc    600
   tgtcgattga ccacaactag gggtttgcgt cgaaaagcaa gcacgcctgg tgcctgattt    660
   gagcggtttt acctatggcg ctttggcgcc gtcaaactgt cccagcgatt tcattattat    720
   tttcgtgcat tcaccgttat agttataggc atg agc aat aaa gag tac cgg ccc     774
                                    Met Ser Asn Lys Glu Tyr Arg Pro
                                    1               5
   aca ctc gcc cag ctt cgc acc ttt gtc acc atc gca gaa tgc aag cac      822
   Thr Leu Ala Gln Leu Arg Thr Phe Val Thr Ile Ala Glu Cys Lys His
       10                  15                  20
   ttt ggt act gct gcc acc aag ctg tcc att tcg cag cca tcc ctc tcc      870
   Phe Gly Thr Ala Ala Thr Lys Leu Ser Ile Ser Gln Pro Ser Leu Ser
   25                  30                  35                  40
   cag gca ctt gtc gca tta gaa aca ggc ctg gga gtt cag ctg att gaa      918
   Gln Ala Leu Val Ala Leu Glu Thr Gly Leu Gly Val Gln Leu Ile Glu
                   45                  50                  55
   cgc tcc acc cgc aag gtc att gtc acc cca gcg ggc gag aag ttg ctg      966
   Arg Ser Thr Arg Lys Val Ile Val Thr Pro Ala Gly Glu Lys Leu Leu
               60                  65                  70
   cca ttc gcc aaa tcc acc ctt gac gcg gcg gag tct ttc ctc tcc cac     1014
   Pro Phe Ala Lys Ser Thr Leu Asp Ala Ala Glu Ser Phe Leu Ser His
           75                  80                  85
   gtc aag ggc gcc aac ggt tcg ctc act gga ccg ttg acc gta ggc atc     1062
   Val Lys Gly Ala Asn Gly Ser Leu Thr Gly Pro Leu Thr Val Gly Ile
       90                  95                  100
   atc ccc acg gcg gct cct tac att ttg ccg tca atg ctg tcc atc gtg      1110
   Ile Pro Thr Ala Ala Pro Tyr Ile Leu Pro Ser Met Leu Ser Ile Val
   105                 110                 115                 120
   gat gaa gaa tat cca gat ctg gaa cct cac atc gtc gag gac caa acc      1158
   Asp Glu Glu Tyr Pro Asp Leu Glu Pro His Ile Val Glu Asp Gln Thr
                   125                 130                 135
   aag cat ctt ctc gcg ttg ctg cgc gac ggc gcc atc gac gtc gcc atg      1206
   Lys His Leu Leu Ala Leu Leu Arg Asp Gly Ala Ile Asp Val Ala Met
               140                 145                 150
   atg gcc ctg cct tct gag gca cca ggc atg aag gaa atc ccc ctc tac      1254
   Met Ala Leu Pro Ser Glu Ala Pro Gly Met Lys Glu Ile Pro Leu Tyr
           155                 160                 165
   gac gaa gac ttt atc gtc gtt aca gct agc gat cac ccc ttc gcc ggc      1302
   Asp Glu Asp Phe Ile Val Val Thr Ala Ser Asp His Pro Phe Ala Gly
       170                 175                 180
   cgc caa gac tta gaa cta tcc gcc tta gaa gac ctc gat ctg ctg ctt      1350
   Arg Gln Asp Leu Glu Leu Ser Ala Leu Glu Asp Leu Asp Leu Leu Leu
   185                 190                 195                 200
   ctc gac gac gga cac tgc ctc cac gac caa att gtg gac ctg tgc cgc      1398
   Leu Asp Asp Gly His Cys Leu His Asp Gln Ile Val Asp Leu Cys Arg
                   205                 210                 215
   cgc gga gac atc aac ccc att agc tcc act act gct gtc acc cgc gca      1446
   Arg Gly Asp Ile Asn Pro Ile Ser Ser Thr Thr Ala Val Thr Arg Ala
               220                 225                 230
   tcc agc ctt acc acc gtc atg cag ctc gtc gtc gcc ggc ctt gga tcc      1494
   Ser Ser Leu Thr Thr Val Met Gln Leu Val Val Ala Gly Leu Gly Ser
           235                 240                 245
   acc ttg gtc cca atc agc gca atc cca tgg gaa tgc acc cga cca gga      1542
   Thr Leu Val Pro Ile Ser Ala Ile Pro Trp Glu Cys Thr Arg Pro Gly
       250                 255                 260
   ctg gca aca gcc aac ttc aac tct gat gtc acc gca aac cgc cgc att      1590
   Leu Ala Thr Ala Asn Phe Asn Ser Asp Val Thr Ala Asn Arg Arg Ile
   265                 270                 275                 280
   gga ttg gtg tac cgt tcc tct tct tct cgc gcc gaa gag ttc gaa cag      1638
   Gly Leu Val Tyr Arg Ser Ser Ser Ser Arg Ala Glu Glu Phe Glu Gln
                   285                 290                 295
   ttt gca ctc att ttg cag cgc gct ttc caa gaa gcc gtc gcg ctt gct      1686
   Phe Ala Leu Ile Leu Gln Arg Ala Phe Gln Glu Ala Val Ala Leu Ala
               300                 305                 310
   gcc tca act ggc atc acc ttg aag caa aat gtc gcg gta gcg cag          1731
   Ala Ser Thr Gly Ile Thr Leu Lys Gln Asn Val Ala Val Ala Gln
           315                 320                 325
   taagtttttc tagaggtttt ccagagtcag ctacaagcaa aaagcccttt ccattgatgc   1791
   acaccaacgt gagattcaag ggaaagggct ttattgattg cagaatgcct actgcattag   1851
   cggcgctcca ccggaatatt tccaccactg atctggcggt aaatatgaac ggtagacagc   1911
   atcattactg gcagcacgat gatcaggccg aaaccgatcg tgaaaatacc gatgatgagt   1971
   gtggcaaacc cgccaaccag ggagaacagc aacaacattg ggtagttgcg cagcgagtcc   2031
   ttgaaaccag tttgaacagc gcttcctgca gtgtggtgac catcagctgt gtagtacacc   2091
   cagtaggagt acagaggatt gatgaagaac aacaccaggt agacgatgaa gaacatgcct   2151
   aaaccgctgt tattgacctc aacggtaccg gcagcgtcat taaacgacac cagattttga   2211
   gtgagaaaag tggtgaaggt gcccaggatg atgccgaaga tacccagccc caccatcaga   2271
   atcactgttt gaccaacatt gatgggttta aagaaatcac cgaagcgaac tttgtgtcca   2331
   tcaacagaaa gcagtgcacc gcgcatgacg caaatggtta ttgcgaaggt gatgattccg   2391
   atagctacgt tcaacagggt ctcggaaacc gaaaaaccag aagtcgtcgt gcccgcatta   2451
   gggtcgatca aaaatgataa gtagccaagc aac                                2484
<210> 8
   <211> 327
   <212> PRT
   <213> Corynebacterium glutamicum
<400> 8
   Met Ser Asn Lys Glu Tyr Arg Pro Thr Leu Ala Gln Leu Arg Thr Phe
   1               5                   10                  15
   Val Thr Ile Ala Glu Cys Lys His Phe Gly Thr Ala Ala Thr Lys Leu
               20                  25                  30
   Ser Ile Ser Gln Pro Ser Leu Ser Gln Ala Leu Val Ala Leu Glu Thr
           35                  40                  45
   Gly Leu Gly Val Gln Leu Ile Glu Arg Ser Thr Arg Lys Val Ile Val
       50                  55                  60
   Thr Pro Ala Gly Glu Lys Leu Leu Pro Phe Ala Lys Ser Thr Leu Asp
   65                  70                  75                  80
   Ala Ala Glu Ser Phe Leu Ser His Val Lys Gly Ala Asn Gly Ser Leu
                   85                  90                  95
   Thr Gly Pro Leu Thr Val Gly Ile Ile Pro Thr Ala Ala Pro Tyr Ile
               100                 105                 110
   Leu Pro Ser Met Leu Ser Ile Val Asp Glu Glu Tyr Pro Asp Leu Glu
           115                 120                 125
   Pro His Ile Val Glu Asp Gln Thr Lys His Leu Leu Ala Leu Leu Arg
       130                 135                 140
   Asp Gly Ala Ile Asp Val Ala Met Met Ala Leu Pro Ser Glu Ala Pro
   145                 150                 155                 160
   Gly Met Lys Glu Ile Pro Leu Tyr Asp Glu Asp Phe Ile Val Val Thr
                   165                 170                 175
   Ala Ser Asp His Pro Phe Ala Gly Arg Gln Asp Leu Glu Leu Ser Ala
               180                 185                 190
   Leu Glu Asp Leu Asp Leu Leu Leu Leu Asp Asp Gly His Cys Leu His
   195 200 205
   Asp Gln Ile Val Asp Leu Cys Arg Arg Gly Asp Ile Asn Pro Ile Ser
   210 215 220
   Ser Thr Thr Ala Val Thr Arg Ala Ser Ser Leu Thr Thr Val Met Gln
   225 230 235 240
   Leu Val Val Ala Gly Leu Gly Ser Thr Leu Val Pro Ile Ser Ala Ile
   245 250 255
   Pro Trp Glu Cys Thr Arg Pro Gly Leu Ala Thr Ala Asn Phe Asn Ser
   260 265 270
   Asp Val Thr Ala Asn Arg Arg Ile Gly Leu Val Tyr Arg Ser Ser Ser
   275 280 285
   Ser Arg Ala Glu Glu Phe Glu Gln Phe Ala Leu Ile Leu Gln Arg Ala
   290 295 300
   Phe Gln Glu Ala Val Ala Leu Ala Ala Ser Thr Gly Ile Thr Leu Lys
   305 310 315 320
   Gln Asn Val Ala Val Ala Gln
   325
<210> 9
   <211> 28
   <212> DNA
   <213> Artificial Sequence
<220>
   <223> Primer oxyR_XL_A1
<220>
   <221> misc_feature
   <222> (1)..(2)
<220>
   <221> misc_structure
   <222> (3)..(8)
   <223> ECoRI-Restriktionsschnittstelle
<400> 9
   gcgaattcgg gcatttacca tcatggtg 28
<210> 10
   <211> 28
   <212> DNA
   <213> Artificial Sequence
<220>
   <223> Primer oxyR_XL_E1
<220> eolf-seql.txt
   <221> misc_feature
   <222> (1)..(2)
<220>
   <221> misc_structure
   <222> (3)..(8)
   <223> EcoRI-Restriktionsschnittstelle
<400> 10
   gcgaattccg ctaatgcagt aggcattc 28
<210> 11
   <211> 1263
   <212> DNA
   <213> Corynebacterium glutamicum
<220>
   <221> CDS
   <222> (1)..(1263)
   <223> lysC-Wildtyp-Gen
<400> 11
   gtg gcc ctg gtc gta cag aaa tat ggc ggt tcc tcg ctt gag agt gcg      48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct      96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat     144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt     192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc     240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg     288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc     336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc     384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc     432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg     480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt     528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag      576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc      624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat      672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg      720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc      768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att      816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct     1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg     1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt     1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca     1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat     1248
   Leu His Glu Gln Phe Gln Leu Gly Gly Glu Asp Glu Ala Val Val Tyr
                   405                 410                 415
   gca ggc acc gga cgc                                                 1263
   Ala Gly Thr Gly Arg
               420
<210> 12
   <211> 421
   <212> PRT
   <213> Corynebacterium glutamicum
<400> 12
   Val Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 13
   <211> 1311
   <212> DNA
   <213> Corynebacterium glutamicum
<220>
   <221> CDS
   <222> (1)..(1308)
   <223> ilvA-Wildtyp-Gen
<400> 13
   atg agt gaa aca tac gtg tct gag aaa agt cca gga gtg atg gct agc       48
   Met Ser Glu Thr Tyr Val Ser Glu Lys Ser Pro Gly Val Met Ala Ser
   1               5                   10                  15
   gga gcg gag ctg att cgt gcc gcc gac att caa acg gcg cag gca cga       96
   Gly Ala Glu Leu Ile Arg Ala Ala Asp Ile Gln Thr Ala Gln Ala Arg
               20                  25                  30
   att tcc tcc gtc att gca cca act cca ttg cag tat tgc cct cgt ctt      144
   Ile Ser Ser Val Ile Ala Pro Thr Pro Leu Gln Tyr Cys Pro Arg Leu
           35                  40                  45
   tct gag gaa acc gga gcg gaa atc tac ctt aag cgt gag gat ctg cag      192
   Ser Glu Glu Thr Gly Ala Glu Ile Tyr Leu Lys Arg Glu Asp Leu Gln
       50                  55                  60
   gat gtt cgt tcc tac aag atc cgc ggt gcg ctg aac tct gga gcg cag      240
   Asp Val Arg Ser Tyr Lys Ile Arg Gly Ala Leu Asn Ser Gly Ala Gln
   65                  70                  75                  80
   ctc acc caa gag cag cgc gat gca ggt atc gtt gcc gca tct gca ggt      288
   Leu Thr Gln Glu Gln Arg Asp Ala Gly Ile Val Ala Ala Ser Ala Gly
                   85                  90                  95
   aac cat gcc cag ggc gtg gcc tat gtg tgc aag tcc ttg ggc gtt cag      336
   Asn His Ala Gln Gly Val Ala Tyr Val Cys Lys Ser Leu Gly Val Gln
               100                 105                 110
   gga cgc atc tat gtt cct gtg cag act cca aag caa aag cgt gac cgc      384
   Gly Arg Ile Tyr Val Pro Val Gln Thr Pro Lys Gln Lys Arg Asp Arg
           115                 120                 125
   atc atg gtt cac ggc gga gag ttt gtc tcc ttg gtg gtc act ggc aat      432
   Ile Met Val His Gly Gly Glu Phe Val Ser Leu Val Val Thr Gly Asn
       130                 135                 140
   aac ttc gac gaa gca tcg gct gca gcg cat gaa gat gca gag cgc acc      480
   Asn Phe Asp Glu Ala Ser Ala Ala Ala His Glu Asp Ala Glu Arg Thr
   145                 150                 155                 160
   ggc gca acg ctg atc gag cct ttc gat gct cgc aac acc gtc atc ggt      528
   Gly Ala Thr Leu Ile Glu Pro Phe Asp Ala Arg Asn Thr Val Ile Gly
                   165                 170                 175
   cag ggc acc gtg gct gct gag atc ttg tcg cag ctg act tcc atg ggc      576
   Gln Gly Thr Val Ala Ala Glu Ile Leu Ser Gln Leu Thr Ser Met Gly
               180                 185                 190
   aag agt gca gat cac gtg atg gtt cca gtc ggc ggt ggc gga ctt ctt      624
   Lys Ser Ala Asp His Val Met Val Pro Val Gly Gly Gly Gly Leu Leu
           195                 200                 205
   gca ggt gtg gtc agc tac atg gct gat atg gca cct cgc act gcg atc      672
   Ala Gly Val Val Ser Tyr Met Ala Asp Met Ala Pro Arg Thr Ala Ile
       210                 215                 220
   gtt ggt atc gaa cca gcg gga gca gca tcc atg cag gct gca ttg cac      720
   Val Gly Ile Glu Pro Ala Gly Ala Ala Ser Met Gln Ala Ala Leu His
   225                 230                 235                 240
   aat ggt gga cca atc act ttg gag act gtt gat ccc ttt gtg gac ggc      768
   Asn Gly Gly Pro Ile Thr Leu Glu Thr Val Asp Pro Phe Val Asp Gly
                   245                 250                 255
   gca gca gtc aaa cgt gtc gga gat ctc aac tac acc atc gtg gag aag      816
   Ala Ala Val Lys Arg Val Gly Asp Leu Asn Tyr Thr Ile Val Glu Lys
               260                 265                 270
   aac cag ggt cgc gtg cac atg atg agc gcg acc gag ggc gct gtg tgt      864
   Asn Gln Gly Arg Val His Met Met Ser Ala Thr Glu Gly Ala Val Cys
           275                 280                 285
   act gag atg ctc gat ctt tac caa aac gaa ggc atc atc gcg gag cct      912
   Thr Glu Met Leu Asp Leu Tyr Gln Asn Glu Gly Ile Ile Ala Glu Pro
       290                 295                 300
   gct ggc gcg ctg tct atc gct ggg ttg aag gaa atg tcc ttt gca cct      960
   Ala Gly Ala Leu Ser Ile Ala Gly Leu Lys Glu Met Ser Phe Ala Pro
   305                 310                 315                 320
   ggt tct gtc gtg gtg tgc atc atc tct ggt ggc aac aac gat gtg ctg     1008
   Gly Ser Val Val Val Cys Ile Ile Ser Gly Gly Asn Asn Asp Val Leu
                   325                 330                 335
   cgt tat gcg gaa atc gct gag cgc tcc ttg gtg cac cgc ggt ttg aag     1056
   Arg Tyr Ala Glu Ile Ala Glu Arg Ser Leu Val His Arg Gly Leu Lys
               340                 345                 350
   cac tac ttc ttg gtg aac ttc ccg caa aag cct ggt cag ttg cgt cac        1104
   His Tyr Phe Leu Val Asn Phe Pro Gln Lys Pro Gly Gln Leu Arg His
           355                 360                 365
   ttc ctg gaa gat atc ctg gga ccg gat gat gac atc acg ctg ttt gag        1152
   Phe Leu Glu Asp Ile Leu Gly Pro Asp Asp Asp Ile Thr Leu Phe Glu
       370                 375                 380
   tac ctc aag cgc aac aac cgt gag acc ggt act gcg ttg gtg ggt att        1200
   Tyr Leu Lys Arg Asn Asn Arg Glu Thr Gly Thr Ala Leu Val Gly Ile
   385                 390                 395                 400
   cac ttg agt gaa gca tca gga ttg gat tct ttg ctg gaa cgt atg gag        1248
   His Leu Ser Glu Ala Ser Gly Leu Asp Ser Leu Leu Glu Arg Met Glu
                   405                 410                 415
   gaa tcg gca att gat tcc cgt cgc ctc gag ccg ggc acc cct gag tac        1296
   Glu Ser Ala Ile Asp Ser Arg Arg Leu Glu Pro Gly Thr Pro Glu Tyr
               420                 425                 430
   gaa tac ttg acc taa                                                    1311
   Glu Tyr Leu Thr
           435
<210> 14
   <211> 436
   <212> PRT
   <213> Corynebacterium glutamicum
<400> 14
   Met Ser Glu Thr Tyr Val Ser Glu Lys Ser Pro Gly Val Met Ala Ser
   1               5                   10                  15
   Gly Ala Glu Leu Ile Arg Ala Ala Asp Ile Gln Thr Ala Gln Ala Arg
               20                  25                  30
   Ile Ser Ser Val Ile Ala Pro Thr Pro Leu Gln Tyr Cys Pro Arg Leu
           35                  40                  45
   Ser Glu Glu Thr Gly Ala Glu Ile Tyr Leu Lys Arg Glu Asp Leu Gln
       50                  55                  60
   Asp Val Arg Ser Tyr Lys Ile Arg Gly Ala Leu Asn Ser Gly Ala Gln
   65                  70                  75                  80
   Leu Thr Gln Glu Gln Arg Asp Ala Gly Ile Val Ala Ala Ser Ala Gly
                   85                  90                  95
   Asn His Ala Gln Gly Val Ala Tyr Val Cys Lys Ser Leu Gly Val Gln
               100                 105                 110
   Gly Arg Ile Tyr Val Pro Val Gln Thr Pro Lys Gln Lys Arg Asp Arg
           115                 120                 125
   Ile Met Val His Gly Gly Glu Phe Val Ser Leu Val Val Thr Gly Asn
       130                 135                 140
   Asn Phe Asp Glu Ala Ser Ala Ala Ala His Glu Asp Ala Glu Arg Thr
   145 150 155 160
   Gly Ala Thr Leu Ile Glu Pro Phe Asp Ala Arg Asn Thr Val Ile Gly
   165 170 175
   Gln Gly Thr Val Ala Ala Glu Ile Leu Ser Gln Leu Thr Ser Met Gly
   180 185 190
   Lys Ser Ala Asp His Val Met Val Pro Val Gly Gly Gly Gly Leu Leu
   195 200 205
   Ala Gly Val Val Ser Tyr Met Ala Asp Met Ala Pro Arg Thr Ala Ile
   210 215 220
   Val Gly Ile Glu Pro Ala Gly Ala Ala Ser Met Gln Ala Ala Leu His
   225 230 235 240
   Asn Gly Gly Pro Ile Thr Leu Glu Thr Val Asp Pro Phe Val Asp Gly
   245 250 255
   Ala Ala Val Lys Arg Val Gly Asp Leu Asn Tyr Thr Ile Val Glu Lys
   260 265 270
   Asn Gln Gly Arg Val His Met Met Ser Ala Thr Glu Gly Ala Val Cys
   275 280 285
   Thr Glu Met Leu Asp Leu Tyr Gln Asn Glu Gly Ile Ile Ala Glu Pro
   290 295 300
   Ala Gly Ala Leu Ser Ile Ala Gly Leu Lys Glu Met Ser Phe Ala Pro
   305 310 315 320
   Gly Ser Val Val Val Cys Ile Ile Ser Gly Gly Asn Asn Asp Val Leu
   325 330 335
   Arg Tyr Ala Glu Ile Ala Glu Arg Ser Leu Val His Arg Gly Leu Lys
   340 345 350
   His Tyr Phe Leu Val Asn Phe Pro Gln Lys Pro Gly Gln Leu Arg His
   355 360 365
   Phe Leu Glu Asp Ile Leu Gly Pro Asp Asp Asp Ile Thr Leu Phe Glu
   370 375 380
   Tyr Leu Lys Arg Asn Asn Arg Glu Thr Gly Thr Ala Leu Val Gly Ile
   385 390 395 400
   His Leu Ser Glu Ala Ser Gly Leu Asp Ser Leu Leu Glu Arg Met Glu
   405 410 415
   Glu Ser Ala Ile Asp Ser Arg Arg Leu Glu Pro Gly Thr Pro Glu Tyr
   420 425 430
   Glu Tyr Leu Thr
   435
<210> 15
   <211> 1599
   <212> DNA
   <213> Corynebacterium glutamicum
<220>
   <221> misc_feature
   <222> (1)..(1599)
   <223> PCR-Produkt
<220>
   <221> misc_feature
   <222> (1)..(2)
<220>
   <221> misc_structure
   <222> (3)..(8)
   <223> EcoRI-Restriktionsschnittstelle
<220>
   <221> CDS
   <222> (489)..(1469)
<220>
   <221> mutation
   <222> (754)..(754)
   <223> C -> Transition
<220>
   <221> misc_feature
   <222> (1219)..(1219)
   <223> Cytosin
<220>
   <221> misc_structure
   <222> (1592)..(1597)
   <223> EcoRI-Restriktionsschnittstelle
<220>
   <221> misc_feature
   <222> (1598)..(1599)
<400> 15
   gcgaattcgg gcatttacca tcatggtgcg caacgccatg ttccgccttg tggagctatt     60
   tgcttatgaa aaggaagatc agcttagtca gatgactgaa tacctggatg aggctcctga    120
   tttcggtgct gcgatggatg cgtactttga tgaatatgcg gatcttgata ccggcccggc    180
   agctcgtgga ccagagttct tcaaggtaga gcacacggga agaatgtggg aggtgcgtca    240
   ggtggtgaag gatccagaag gtgataattc cttcgcgttt gttgccacca ttgatcttga    300
   tgcctctgat gatgcaggtg aggtgcgttt tggatcgctg tcgattgacc acaactaggg    360
   gtttgcgtcg aaaagcaagc acgcctggtg cctgatttga gcggttttac ctatggcgct    420
   ttggcgccgt caaactgtcc cagcgatttc attattattt tcgtgcattc accgttatag    480
   ttataggc atg agc aat aaa gag tac cgg ccc aca ctc gcc cag ctt cgc 530
   Met Ser Asn Lys Glu Tyr Arg Pro Thr Leu Ala Gln Leu Arg
   1 5 10
   acc ttt gtc acc atc gca gaa tgc aag cac ttt ggt act gct gcc acc 578
   Thr Phe Val Thr Ile Ala Glu Cys Lys His Phe Gly Thr Ala Ala Thr
   15 20 25 30
   aag ctg tcc att tcg cag cca tcc ctc tcc cag gca ctt gtc gca tta 626
   Lys Leu Ser Ile Ser Gln Pro Ser Leu Ser Gln Ala Leu Val Ala Leu
   35 40 45
   gaa aca ggc ctg gga gtt cag ctg att gaa cgc tcc acc cgc aag gtc 674
   Glu Thr Gly Leu Gly Val Gln Leu Ile Glu Arg Ser Thr Arg Lys Val
   50 55 60
   att gtc acc cca gcg ggc gag aag ttg ctg cca ttc gcc aaa tcc acc 722
   Ile Val Thr Pro Ala Gly Glu Lys Leu Leu Pro Phe Ala Lys Ser Thr
   65 70 75
   ctt gac gcg gcg gag tct ttc ctc tcc cac gtc aag ggc gcc aac ggt 770
   Leu Asp Ala Ala Glu Ser Phe Leu Ser His Val Lys Gly Ala Asn Gly
   80 85 90
   tcg ctc act gga ccg ttg acc gta ggc atc atc ccc acg gcg gct cct 818
   Ser Leu Thr Gly Pro Leu Thr Val Gly Ile Ile Pro Thr Ala Ala Pro
   95 100 105 110
   tac att ttg ccg tca atg ctg tcc atc gtg gat gaa gaa tat cca gat 866
   Tyr Ile Leu Pro Ser Met Leu Ser Ile Val Asp Glu Glu Tyr Pro Asp
   115 120 125
   ctg gaa cct cac atc gtc gag gac caa acc aag cat ctt ctc gcg ttg 914
   Leu Glu Pro His Ile Val Glu Asp Gln Thr Lys His Leu Leu Ala Leu
   130 135 140
   ctg cgc gac ggc gcc atc gac gtc gcc atg atg gcc ctg cct tct gag 962
   Leu Arg Asp Gly Ala Ile Asp Val Ala Met Met Ala Leu Pro Ser Glu
   145 150 155
   gca cca ggc atg aag gaa atc ccc ctc tac gac gaa gac ttt atc gtc 1010
   Ala Pro Gly Met Lys Glu Ile Pro Leu Tyr Asp Glu Asp Phe Ile Val
   160 165 170
   gtt aca gct agc gat cac ccc ttc gcc ggc cgc caa gac tta gaa cta 1058
   Val Thr Ala Ser Asp His Pro Phe Ala Gly Arg Gln Asp Leu Glu Leu
   175 180 185 190
   tcc gcc tta gaa gac ctc gat ctg ctg ctt ctc gac gac gga cac tgc 1106
   Ser Ala Leu Glu Asp Leu Asp Leu Leu Leu Leu Asp Asp Gly His Cys
   195 200 205
   ctc cac gac caa att gtg gac ctg tgc cgc cgc gga gac atc aac ccc 1154
   Leu His Asp Gln Ile Val Asp Leu Cys Arg Arg Gly Asp Ile Asn Pro
   210 215 220
   att agc tcc act act gct gtc acc cgc gca tcc agc ctt acc acc gtc 1202
   Ile Ser Ser Thr Thr Ala Val Thr Arg Ala Ser Ser Leu Thr Thr Val
   225 230 235
   atg cag ctc gtc gtc gcc ggc ctt gga tcc acc ttg gtc cca atc agc 1250
   Met Gln Leu Val Val Ala Gly Leu Gly Ser Thr Leu Val Pro Ile Ser
   240 245 250
   gca atc cca tgg gaa tgc acc cga cca gga ctg gca aca gcc aac ttc 1298
   Ala Ile Pro Trp Glu Cys Thr Arg Pro Gly Leu Ala Thr Ala Asn Phe
   255 260 265 270
   aac tct gat gtc acc gca aac cgc cgc att gga ttg gtg tac cgt tcc 1346
   Asn Ser Asp Val Thr Ala Asn Arg Arg Ile Gly Leu Val Tyr Arg Ser
                   275                 280                 285
   tct tct tct cgc gcc gaa gag ttc gaa cag ttt gca ctc att ttg cag      1394
   Ser Ser Ser Arg Ala Glu Glu Phe Glu Gln Phe Ala Leu Ile Leu Gln
               290                 295                 300
   cgc gct ttc caa gaa gcc gtc gcg ctt gct gcc tca act ggc atc acc      1442
   Arg Ala Phe Gln Glu Ala Val Ala Leu Ala Ala Ser Thr Gly Ile Thr
           305                 310                 315
   ttg aag caa aat gtc gcg gta gcg cag taagtttttc tagaggtttt            1489
   Leu Lys Gln Asn Val Ala Val Ala Gln
       320                 325
   ccagagtcag ctacaagcaa aaagcccttt ccattgatgc acaccaacgt gagattcaag    1549
   ggaaagggct ttattgattg cagaatgcct actgcattag cggaattcgc               1599
<210> 16
   <211> 327
   <212> PRT
   <213> Corynebacterium glutamicum
<400> 16
   Met Ser Asn Lys Glu Tyr Arg Pro Thr Leu Ala Gln Leu Arg Thr Phe
   1               5                   10                  15
   Val Thr Ile Ala Glu Cys Lys His Phe Gly Thr Ala Ala Thr Lys Leu
               20                  25                  30
   Ser Ile Ser Gln Pro Ser Leu Ser Gln Ala Leu Val Ala Leu Glu Thr
           35                  40                  45
   Gly Leu Gly Val Gln Leu Ile Glu Arg Ser Thr Arg Lys Val Ile Val
       50                  55                  60
   Thr Pro Ala Gly Glu Lys Leu Leu Pro Phe Ala Lys Ser Thr Leu Asp
   65                  70                  75                  80
   Ala Ala Glu Ser Phe Leu Ser His Val Lys Gly Ala Asn Gly Ser Leu
                   85                  90                  95
   Thr Gly Pro Leu Thr Val Gly Ile Ile Pro Thr Ala Ala Pro Tyr Ile
               100                 105                 110
   Leu Pro Ser Met Leu Ser Ile Val Asp Glu Glu Tyr Pro Asp Leu Glu
           115                 120                 125
   Pro His Ile Val Glu Asp Gln Thr Lys His Leu Leu Ala Leu Leu Arg
       130                 135                 140
   Asp Gly Ala Ile Asp Val Ala Met Met Ala Leu Pro Ser Glu Ala Pro
   145                 150                 155                 160
   Gly Met Lys Glu Ile Pro Leu Tyr Asp Glu Asp Phe Ile Val Val Thr
                   165                 170                 175
   Ala Ser Asp His Pro Phe Ala Gly Arg Gln Asp Leu Glu Leu Ser Ala
               180                 185                 190
   Leu Glu Asp Leu Asp Leu Leu Leu Leu Asp Asp Gly His Cys Leu His
           195                 200                 205
   Asp Gln Ile Val Asp Leu Cys Arg Arg Gly Asp Ile Asn Pro Ile Ser
       210                 215                 220
   Ser Thr Thr Ala Val Thr Arg Ala Ser Ser Leu Thr Thr Val Met Gln
   225                 230                 235                 240
   Leu Val Val Ala Gly Leu Gly Ser Thr Leu Val Pro Ile Ser Ala Ile
                   245                 250                 255
   Pro Trp Glu Cys Thr Arg Pro Gly Leu Ala Thr Ala Asn Phe Asn Ser
               260                 265                 270
   Asp Val Thr Ala Asn Arg Arg Ile Gly Leu Val Tyr Arg Ser Ser Ser
           275                 280                 285
   Ser Arg Ala Glu Glu Phe Glu Gln Phe Ala Leu Ile Leu Gln Arg Ala
       290                 295                 300
   Phe Gln Glu Ala Val Ala Leu Ala Ala Ser Thr Gly Ile Thr Leu Lys
   305                 310                 315                 320
   Gln Asn Val Ala Val Ala Gln
                   325
<210> 17
   <211> 117
   <212> DNA
   <213> Corynebacterium glutamicum
<220>
   <221> CDS
   <222> (1)..(117)
   <223> Teil der Kodierregion des oxyR-Allels
<220>
   <221> misc_feature
   <222> (58)..(60)
   <223> n is a, c, g, or t
<400> 17
   aag ttg ctg cca ttc gcc aaa tcc acc ctt gac gcg gcg gag tct ttc      48
   Lys Leu Leu Pro Phe Ala Lys Ser Thr Leu Asp Ala Ala Glu Ser Phe
   1               5                   10                  15
   ctc tcc cac nnn aag ggc gcc aac ggt tcg ctc act gga ccg ttg acc      96
   Leu Ser His Xaa Lys Gly Ala Asn Gly Ser Leu Thr Gly Pro Leu Thr
               20                  25                  30
   gta ggc atc atc ccc acg gcg                                          117
   Val Gly Ile Ile Pro Thr Ala
                   35
<210> 18
   <211> 39
   <212> PRT
   <213> Corynebacterium glutamicum
<220>
   <221> misc_feature
   <222> (20)..(20)
   <223> The 'Xaa' at location 20 stands for Lys, Asn, Arg, ser, Thr, Ile, Met, Glu, Asp, Gly, Val, Gln, His, Pro, Leu, Tyr, Trp, Cys, or Phe.
<400> 18
   Lys Leu Leu Pro Phe Ala Lys Ser Thr Leu Asp Ala Ala Glu Ser Phe
   1               5                   10                  15
   Leu Ser His Xaa Lys Gly Ala Asn Gly Ser Leu Thr Gly Pro Leu Thr
               20                  25                  30
   Val Gly Ile Ile Pro Thr Ala
           35
<210> 19
   <211> 117
   <212> DNA
   <213> Corynebacterium glutamicum
<220>
   <221> CDS
   <222> (1)..(117)
   <223> Teil der Kodierregion des oxyR-Allels
<400> 19
   aag ttg ctg cca ttc gcc aaa tcc acc ctt gac gcg gcg gag tct ttc      48
   Lys Leu Leu Pro Phe Ala Lys Ser Thr Leu Asp Ala Ala Glu Ser Phe
   1               5                   10                  15
   ctc tcc cac gtc aag ggc gcc aac ggt tcg ctc act gga ccg ttg acc      96
   Leu Ser His Val Lys Gly Ala Asn Gly Ser Leu Thr Gly Pro Leu Thr
               20                  25                  30
   gta ggc atc atc ccc acg gcg                                          117
   Val Gly Ile Ile Pro Thr Ala
           35
<210> 20
   <211> 39
   <212> PRT
   <213> Corynebacterium glutamicum
<400> 20
   Lys Leu Leu Pro Phe Ala Lys Ser Thr Leu Asp Ala Ala Glu Ser Phe
   1               5                   10                  15
   Leu Ser His Val Lys Gly Ala Asn Gly Ser Leu Thr Gly Pro Leu Thr
               20                  25                  30
   Val Gly Ile Ile Pro Thr Ala
           35
<210> 21
   <211> 984
   <212> DNA
   <213> Corynebacterium glutamicum
<220>
   <221> CDS
   <222> (1)..(981)
   <223> oxyR-Allel
<220>
   <221> mutation
   <222> (266)..(266)
   <223> C -> T Transition
<220>
   <221> mutation
   <222> (731)..(731)
   <223> C -> T Transition
<400> 21
   atg agc aat aaa gag tac cgg ccc aca ctc gcc cag ctt cgc acc ttt      48
   Met Ser Asn Lys Glu Tyr Arg Pro Thr Leu Ala Gln Leu Arg Thr Phe
   1               5                   10                  15
   gtc acc atc gca gaa tgc aag cac ttt ggt act gct gcc acc aag ctg      96
   Val Thr Ile Ala Glu Cys Lys His Phe Gly Thr Ala Ala Thr Lys Leu
               20                  25                  30
   tcc att tcg cag cca tcc ctc tcc cag gca ctt gtc gca tta gaa aca     144
   Ser Ile Ser Gln Pro Ser Leu Ser Gln Ala Leu Val Ala Leu Glu Thr
           35                  40                  45
   ggc ctg gga gtt cag ctg att gaa cgc tcc acc cgc aag gtc att gtc     192
   Gly Leu Gly Val Gln Leu Ile Glu Arg Ser Thr Arg Lys Val Ile Val
       50                  55                  60
   acc cca gcg ggc gag aag ttg ctg cca ttc gcc aaa tcc acc ctt gac     240
   Thr Pro Ala Gly Glu Lys Leu Leu Pro Phe Ala Lys Ser Thr Leu Asp
   65                  70                  75                  80
   gcg gcg gag tct ttc ctc tcc cac gtc aag ggc gcc aac ggt tcg ctc     288
   Ala Ala Glu Ser Phe Leu Ser His Val Lys Gly Ala Asn Gly Ser Leu
                   85                  90                  95
   act gga ccg ttg acc gta ggc atc atc ccc acg gcg gct cct tac att     336
   Thr Gly Pro Leu Thr Val Gly Ile Ile Pro Thr Ala Ala Pro Tyr Ile
               100                 105                 110
   ttg ccg tca atg ctg tcc atc gtg gat gaa gaa tat cca gat ctg gaa     384
   Leu Pro Ser Met Leu Ser Ile Val Asp Glu Glu Tyr Pro Asp Leu Glu
           115                 120                 125
   cct cac atc gtc gag gac caa acc aag cat ctt ctc gcg ttg ctg cgc     432
   Pro His Ile Val Glu Asp Gln Thr Lys His Leu Leu Ala Leu Leu Arg
       130                 135                 140
   gac ggc gcc atc gac gtc gcc atg atg gcc ctg cct tct gag gca cca     480
   Asp Gly Ala Ile Asp Val Ala Met Met Ala Leu Pro Ser Glu Ala Pro
   145                 150                 155                 160
   ggc atg aag gaa atc ccc ctc tac gac gaa gac ttt atc gtc gtt aca     528
   Gly Met Lys Glu Ile Pro Leu Tyr Asp Glu Asp Phe Ile Val Val Thr
                   165                 170                 175
   gct agc gat cac ccc ttc gcc ggc cgc caa gac tta gaa cta tcc gcc      576
   Ala Ser Asp His Pro Phe Ala Gly Arg Gln Asp Leu Glu Leu Ser Ala
               180                 185                 190
   tta gaa gac ctc gat ctg ctg ctt ctc gac gac gga cac tgc ctc cac      624
   Leu Glu Asp Leu Asp Leu Leu Leu Leu Asp Asp Gly His Cys Leu His
           195                 200                 205
   gac caa att gtg gac ctg tgc cgc cgc gga gac atc aac ccc att agc      672
   Asp Gln Ile Val Asp Leu Cys Arg Arg Gly Asp Ile Asn Pro Ile Ser
       210                 215                 220
   tcc act act gct gtc acc cgc gca tcc agc ctt acc acc gtc atg cag      720
   Ser Thr Thr Ala Val Thr Arg Ala Ser Ser Leu Thr Thr Val Met Gln
   225                 230                 235                 240
   ctc gtc gtc gtc ggc ctt gga tcc acc ttg gtc cca atc agc gca atc      768
   Leu Val Val Val Gly Leu Gly Ser Thr Leu Val Pro Ile Ser Ala Ile
                   245                 250                 255
   cca tgg gaa tgc acc cga cca gga ctg gca aca gcc aac ttc aac tct      816
   Pro Trp Glu Cys Thr Arg Pro Gly Leu Ala Thr Ala Asn Phe Asn Ser
               260                 265                 270
   gat gtc acc gca aac cgc cgc att gga ttg gtg tac cgt tcc tct tct      864
   Asp Val Thr Ala Asn Arg Arg Ile Gly Leu Val Tyr Arg Ser Ser Ser
           275                 280                 285
   tct cgc gcc gaa gag ttc gaa cag ttt gca ctc att ttg cag cgc gct      912
   Ser Arg Ala Glu Glu Phe Glu Gln Phe Ala Leu Ile Leu Gln Arg Ala
       290                 295                 300
   ttc caa gaa gcc gtc gcg ctt gct gcc tca act ggc atc acc ttg aag      960
   Phe Gln Glu Ala Val Ala Leu Ala Ala Ser Thr Gly Ile Thr Leu Lys
   305                 310                 315                 320
   caa aat gtc gcg gta gcg cag taa                                      984
   Gln Asn Val Ala Val Ala Gln
                   325
<210> 22
   <211> 327
   <212> PRT
   <213> Corynebacterium glutamicum
<400> 22
   Met Ser Asn Lys Glu Tyr Arg Pro Thr Leu Ala Gln Leu Arg Thr Phe
   1               5                   10                  15
   Val Thr Ile Ala Glu Cys Lys His Phe Gly Thr Ala Ala Thr Lys Leu
               20                  25                  30
   Ser Ile Ser Gln Pro Ser Leu Ser Gln Ala Leu Val Ala Leu Glu Thr
           35                  40                  45
   Gly Leu Gly Val Gln Leu Ile Glu Arg Ser Thr Arg Lys Val Ile Val
       50                  55                  60
   Thr Pro Ala Gly Glu Lys Leu Leu Pro Phe Ala Lys Ser Thr Leu Asp
   65                  70                  75                  80
   Ala Ala Glu Ser Phe Leu Ser His Val Lys Gly Ala Asn Gly Ser Leu
   85 90 95
   Thr Gly Pro Leu Thr Val Gly Ile Ile Pro Thr Ala Ala Pro Tyr Ile
   100 105 110
   Leu Pro Ser Met Leu Ser Ile Val Asp Glu Glu Tyr Pro Asp Leu Glu
   115 120 125
   Pro His Ile Val Glu Asp Gln Thr Lys His Leu Leu Ala Leu Leu Arg
   130 135 140
   Asp Gly Ala Ile Asp Val Ala Met Met Ala Leu Pro Ser Glu Ala Pro
   145 150 155 160
   Gly Met Lys Glu Ile Pro Leu Tyr Asp Glu Asp Phe Ile Val Val Thr
   165 170 175
   Ala Ser Asp His Pro Phe Ala Gly Arg Gln Asp Leu Glu Leu Ser Ala
   180 185 190
   Leu Glu Asp Leu Asp Leu Leu Leu Leu Asp Asp Gly His Cys Leu His
   195 200 205
   Asp Gln Ile Val Asp Leu Cys Arg Arg Gly Asp Ile Asn Pro Ile Ser
   210 215 220
   Ser Thr Thr Ala Val Thr Arg Ala Ser Ser Leu Thr Thr Val Met Gln
   225 230 235 240
   Leu Val Val Val Gly Leu Gly Ser Thr Leu Val Pro Ile Ser Ala Ile
   245 250 255
   Pro Trp Glu Cys Thr Arg Pro Gly Leu Ala Thr Ala Asn Phe Asn Ser
   260 265 270
   Asp Val Thr Ala Asn Arg Arg Ile Gly Leu Val Tyr Arg Ser Ser Ser
   275 280 285
   Ser Arg Ala Glu Glu Phe Glu Gln Phe Ala Leu Ile Leu Gln Arg Ala
   290 295 300
   Phe Gln Glu Ala Val Ala Leu Ala Ala Ser Thr Gly Ile Thr Leu Lys
   305 310 315 320
   Gln Asn Val Ala Val Ala Gln
   325
<210> 23
   <211> 698
   <212> DNA
   <213> Corynebacterium glutamicum
<220>
   <221> misc_feature
   <222> (167)..(179)
<220>
   <221> CDS
   <222> (201)..(695)
   <223> dps-wildtyp-Gen
<400> 23
   tcttggtgag ttccccagct agtcgatgaa tcacgtgtcc ttcaggcata atcaccaacc      60
   ttagtagccg gtgtgccgat ttgataaaaa actaagcgtg gcctgcggga atcggcactt     120
   tcaggatagg acaacctaat ataaataagc ttaggctaag ggccggtgac aatttatcaa     180
   gcagtgctat aataggggtc atg gca aac tac aca gtc cct gga atc aac gag     233
                         Met Ala Asn Tyr Thr Val Pro Gly Ile Asn Glu
                         1               5                   10
   aat gac gca aag cag ctt att gat gga ctg cag gag cgt ctc acc gac       281
   Asn Asp Ala Lys Gln Leu Ile Asp Gly Leu Gln Glu Arg Leu Thr Asp
               15                  20                  25
   tac aac gat ctt cac ctc atc ttg aag cac gtg cac tgg aac gtc act       329
   Tyr Asn Asp Leu His Leu Ile Leu Lys His Val His Trp Asn Val Thr
           30                  35                  40
   ggc ccc aac ttc att gct gtt cac gaa atg ctc gac cca cag gtt gac       377
   Gly Pro Asn Phe Ile Ala Val His Glu Met Leu Asp Pro Gln Val Asp
       45                  50                  55
   ctt gtt cgt ggc tat gct gac gaa gtt gca gag cgc att tcc acc ctc       425
   Leu Val Arg Gly Tyr Ala Asp Glu Val Ala Glu Arg Ile Ser Thr Leu
   60                  65                  70                  75
   gga ggc gca cca gtt gga acc cca gaa ggc cac gtt gct gac cgc acc       473
   Gly Gly Ala Pro Val Gly Thr Pro Glu Gly His Val Ala Asp Arg Thr
                   80                  85                  90
   cca ctg caa tat gag cgc aat gcc gga aat gtc caa gca cac ctc act       521
   Pro Leu Gln Tyr Glu Arg Asn Ala Gly Asn Val Gln Ala His Leu Thr
               95                  100                 105
   gac ctc aat cgc gtg tac acc caa gtg ctg acc gga gtt cgc gag tcc       569
   Asp Leu Asn Arg Val Tyr Thr Gln Val Leu Thr Gly Val Arg Glu Ser
           110                 115                 120
   atg gca tca gcc ggc cca gtg gat cca gta act gaa gac atc tac atc       617
   Met Ala Ser Ala Gly Pro Val Asp Pro Val Thr Glu Asp Ile Tyr Ile
       125                 130                 135
   agc cag gcc gcg gag ctg gag aaa ttc cag tgg ttc atc cgc gca cac       665
   Ser Gln Ala Ala Glu Leu Glu Lys Phe Gln Trp Phe Ile Arg Ala His
   140                 145                 150                 155
   att gtt gat gta gac gga aac atc caa gag taa                           698
   Ile Val Asp Val Asp Gly Asn Ile Gln Glu
                   160                 165
<210> 24
   <211> 165
   <212> PRT
   <213> Corynebacterium glutamicum
<400> 24
   Met Ala Asn Tyr Thr Val Pro Gly Ile Asn Glu Asn Asp Ala Lys Gln
   1               5                   10                  15
   Leu Ile Asp Gly Leu Gln Glu Arg Leu Thr Asp Tyr Asn Asp Leu His
               20                  25                  30
   Leu Ile Leu Lys His Val His Trp Asn Val Thr Gly Pro Asn Phe Ile
           35                  40                  45
   Ala Val His Glu Met Leu Asp Pro Gln Val Asp Leu Val Arg Gly Tyr
       50                  55                  60
   Ala Asp Glu Val Ala Glu Arg Ile Ser Thr Leu Gly Gly Ala Pro Val
   65                  70                  75                  80
   Gly Thr Pro Glu Gly His Val Ala Asp Arg Thr Pro Leu Gln Tyr Glu
                   85                  90                  95
   Arg Asn Ala Gly Asn Val Gln Ala His Leu Thr Asp Leu Asn Arg Val
               100                 105                 110
   Tyr Thr Gln Val Leu Thr Gly Val Arg Glu Ser Met Ala Ser Ala Gly
           115                 120                 125
   Pro Val Asp Pro Val Thr Glu Asp Ile Tyr Ile Ser Gln Ala Ala Glu
       130                 135                 140
   Leu Glu Lys Phe Gln Trp Phe Ile Arg Ala His Ile Val Asp Val Asp
   145                 150                 155                 160
   Gly Asn Ile Gln Glu
                   165